(19) Europäisches Patentamt
European Patent Office
Office européen des brevets

(11) Veröffentlichungsnummer : **0 327 912 B1**

(12) # EUROPÄISCHE PATENTSCHRIFT

(45) Veröffentlichungstag der Patentschrift : **08.04.92 Patentblatt 92/15**

(51) Int. Cl.$^5$ : **C07D 295/00**

(21) Anmeldenummer : **89101520.8**

(22) Anmeldetag : **30.01.89**

(54) **N,N'-disubstituierte Piperazine.**

(30) Priorität : **09.02.88 DE 3803860**

(43) Veröffentlichungstag der Anmeldung : **16.08.89 Patentblatt 89/33**

(45) Bekanntmachung des Hinweises auf die Patenterteilung : **08.04.92 Patentblatt 92/15**

(84) Benannte Vertragsstaaten : **AT BE CH DE ES FR GB GR IT LI NL SE**

(56) Entgegenhaltungen :
**AT-B- 227 269**
**DE-A- 2 727 482**
**DE-B- 1 108 226**
**GB-A- 998 509**

(73) Patentinhaber : **BASF Aktiengesellschaft**
**Carl-Bosch-Strasse 38**
**W-6700 Ludwigshafen (DE)**

(72) Erfinder : **Zipplies, Matthias, Dr.**
**Kastanienweg 1**
**W-6945 Hirschberg (DE)**
Erfinder : **Sauter, Hubert, Dr.**
**Neckarpromenade 20**
**W-6800 Mannheim 1 (DE)**
Erfinder : **Roehl, Franz, Dr.**
**Karl-Otto-Braun-Strasse 8**
**W-6700 Ludwigshafen (DE)**
Erfinder : **Kuekenhoehner, Thomas, Dr.**
**Seidelstrasse 2**
**W-6710 Frankenthal (DE)**
Erfinder : **Janitschke, Lothar, Dr.**
**Wormser Gasse 9 a**
**W-6711 Kleinniedesheim (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

EP 0 327 912 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N,N′-disubstituierte Piperazine, Verfahren zu deren Herstellung, deren Verwendung als Fungizide, fungizide Mittel, die die neuen Wirkstoffe enthalten, sowie Verfahren zur Bekämpfung von Schadpilzen mit diesen Wirkstoffen.

N,N′-Alkyl- oder Aryl-disubstituierte Piperazinderivate, sind als pharmakologisch wirksame Mittel sowie als Futterzusatzstoffe bekannt (GB 850 662, US 2 943 090, BE 588 826, US 2 927 924, DE-28 09 209, BE 853 899, BE 840 326, DE-22 63 211, DE-20 50 684, BE 756 127, US 4 523 014).

Aus DE 30 28 483 sind N,N′-Dialkylpiperazine der Formel V

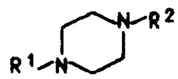

(V)

mit $R^1$ = $C_1$-$C_{12}$-Alkyl, Cycloalkyl, Hydroxyalkyl und $R^2$ = mit Alkyl oder Cycloalkyl substituiertes $C_6$-$C_{20}$-Aryl, als Verbindungen, die das Ausbleichen von Diazotypien verhindern, bekannt.

Aus DE-2 727 482 ist das Dialkylpiperazin-Derivat VI als Verbindung mit fungizider Wirkung bekannt.

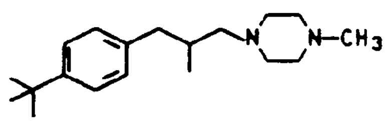

(VI)

Der vorliegenden Erfindung lag die Aufgabe zugrunde, neue Verbindungen dieses Typs mit verbesserten Eigenschaften als Fungizide bereitzustellen.

Es wurde nun gefunden, daß N,N-disubstituierte Piperazine der Formel I

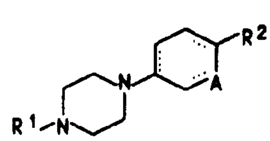

in der die Gruppe ═ ungesättigt (=) oder gesättigt (-) ist und die Substituenten $R^1$, $R^2$ und A die folgende Bedeutung haben:

A = -, -CH=, $-CH_2-$, $-CH_2-CH_2-$,

$R^1$ verzweigtes oder unverzweigtes $C_2$-$C_{20}$-Alkyl, $C_3$-$C_{20}$-Alkenyl, $C_3$-$C_{20}$-Alkinyl, $C_4$-$C_{12}$-Cycloalkyl, $C_4$-$C_{12}$-Cycloalkenyl, $C_4$-$C_{20}$-Alkyl-cycloalkyl, $C_4$-$C_{20}$-Cycloalkenyl-alkyl, $C_4$-$C_{20}$-Alkyl-cycloalkenyl, $C_9$-$C_{11}$-Bicycloalkyl, $C_{10}$-$C_{15}$-Bicycloalkylalkyl, $C_{10}$-$C_{15}$-Alkyl-bicycloalkyl, wobei diese Reste gegebenenfalls einfach bis dreifach durch Hydroxy, 1-3 Halogenatome wie Chlor, Brom, Fluor, $C_1$-$C_5$-Alkoxy oder $C_3$-$C_9$ Trialkylsilyl substituiert sind,

$R^1$ ferner 5- bis 7-gliedriges Heterocycloalkyl mit 1 oder 2 Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl einfach bis dreifach substituiertes 5 bis 7-gliedriges Heterocycloalkylmethyl mit 1 oder 2 Heteroatomen aus der Gruppe Sauerstoff und/oder Schwefel,

$R^2$ verzweigtes $C_3$-$C_{10}$-Alkyl, verzweigtes $C_3$-$C_8$-Alkoxy, $C_3$-$C_7$-Cycloalkyl, Trimethylsilyl, $C_3$-$C_8$-Alkylthio und deren pflanzenphysiologisch verträglichen Salze sich bei guter Pflanzenverträglichkeit hervorragend als Fungizide eignen.

Unter Salzen der Piperazine sind Salze mit beliebigen anorganischen und organischen Säuren zu verstehen, z.B. mit Chlorwasserstoff, Bromwasserstoff, Schwefelsäure, Phosphorsäure, Dodecylbenzolsulfonsäure, Ameisensäure, Essigsäure, Propionsäure, Palmitinsäure, Perfluorheptansäure, Oxalsäure, Malonsäure, Benzoesäure, Äpfelsäure, Dodecylsulfonsäure.

Die neuen N-substituierten N,N′-disubstituierten-Piperazine der Formel I und deren pflanzenphysiologisch verträglichen Salze enthalten chirale Zentren. Sie werden im allgemeinen als Racemate oder gegebenenfalls

als Diastereomerengemische erhalten.

Diastereomerenreine Verbindungen lassen sich bei einigen der neuen Verbindungen beispielsweise durch Destillation, Säulenchromatographie oder aufgrund von Löslichkeitsunterschieden isolieren. Enantiomerenreine Verbindungen lassen sich z.B. durch Racematspaltung mit einem chiralen Hilfsreagenz nach bekannten Methoden beispielsweise über diastereomere Salze erhalten. Für die Anwendung der neuen N,N'-disubstituierten Piperazine der Formel I und deren pflanzenphysiologisch verträglichen Salze als Fungizide sind sowohl die Diastereomeren bzw. die Enantiomeren als auch deren bei der Synthese anfallenden Stereoisomerengemische geeignet. Alle diese Verbindungen werden von der vorliegenden Erfindung umfaßt.

Unter den Verbindungen I sind diejenigen bevorzugt, in denen A die oben genannten Bedeutungen hat, so daß die Gruppe

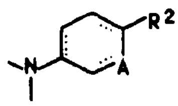

beispielsweise die folgenden Bedeutungen hat:

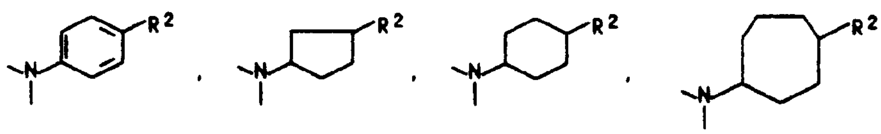

und der Rest $R^1$

$C_2$-$C_{20}$ Alkyl, besonders $C_3$-$C_{19}$-Alkyl, wie beispielsweise Propyl, Isopropyl, Butyl, Isobutyl, But-2-yl, tert.-Butyl. Pentyl, Pent-2-yl, Pent-3-yl, 1,2-Dimethyl-propyl, 2,2-Dimethyl-propyl, Hexyl, Hex-2-yl, Hex-3-yl, 2,3,3-Trimethyl-but-2-yl, 4-Methyl-pent-2-yl, 4-Methyl-pentyl, 3,3-Dimethylbutyl, Heptyl, Hept-2-yl, Hept-3-yl, Hept-4-yl, Diisopropyl-methyl, 1,4-Dimethyl-pentyl, 4,4-Dimethyl-pentyl, Octyl, 2-Methyl-hept-3-yl, 5-Methyl-hept-3-yl, Oct-2-yl, Oct-3-yl, Oct-4-yl, 5,5-Dimethyl-hexyl, 2,4,4-Trimethyl-pentyl, 6-Methyl-hept-2-yl, Nonyl, Non-2-yl, Non-3-yl, Non-4-yl, Non-5-yl, 2,5,5-Trimethyl-hexyl, 2,6-Dimethyl-hept-4-yl, 3,5,5-Trimethyl-hexyl, Decyl, Dec-2-yl, Dec-3-yl, Dec-4-yl, 2,3,5,5-Tetramethyl-hexyl, Undecyl, Dodecyl, Tridecyl, 1,5,9-Trimethyl-decyl, Tetradecyl.

$C_2$-$C_{20}$-Hydroxyalkyl, besonders $C_2$-$C_8$-Hydroxyalkyl, wie beispielsweise Hydroxyethyl, Hydroxypropyl, Hydroxybutyl, Hydroxypentyl, Hydroxyhexyl, Hydroxyheptyl, 1-Hydroxy-but-2-yl, 2-Hydroxy-but-3-yl,

$C_2$-$C_{20}$-Halogenalkyl mit 1-3 Halogenatomen wie Chlor, Brom, Fluor, besonders $C_2$-$C_{10}$-Halogenalkyl mit 1-3 Halogenatomen wie Chlor, Brom, Fluor, wie beispielsweise 3-Chlorpropyl, 6-Chlorhexyl, Trifluorethyl, Trichlorethyl, 5-Chlor-pent-2-yl, 3-Chlor-but-2-yl, 3,3-Dichlor-prop-2-yl,

$C_1$-$C_5$-alkoxy-$C_2$-$C_{20}$-Alkyl, besonders $C_2$-$C_4$-Alkoxy-$C_2$-$C_{10}$-Alkyl, wie beispielsweise Methoxyethyl, Ethoxyethyl, tert.-Butoxyethyl, 3-tert.-Butoxy-propyl, 4-tert.-Butoxy-butyl, Methoxypropyl, Methoxybutyl, Methoxypentyl, Methoxyhexyl, Ethoxyhexyl, 3-Methoxy-prop-2-yl,

$C_3$-$C_9$-Trialkylsilyl-$C_2$-$C_{20}$-Alkyl, besonders $C_3$-$C_6$-Trialkylsilyl-, $C_2$-$C_{10}$-Alkyl, wie beispielsweise Trimethylsilyl-ethyl, Trimethylsilyl-propyl, Trimethylsilyl-butyl, Trimethylsilyl-pentyl, 6-Trimethylsilyl-hexyl,

$C_3$-$C_{20}$-Alkenyl, besonders $C_3$-$C_{14}$-Alkenyl, wie beispielsweise Allyl, Methallyl, Dimethylallyl, Hexenyl, 1,5,9-Trimethyl-decadienyl,

$C_3$-$C_{20}$-Alkinyl, besonders $C_3$-$C_{14}$-Alkinyl, wie beispielsweise Propargyl, 4,4-Dimethyl-2-butin-1-yl,

$C_4$-$C_{12}$-Cycloalkyl, wie beispielsweise Cyclobutyl, Cyclopentyl, Cyclohexyl, Cycloheptyl, Cyclododecyl,

$C_4$-$C_{12}$-Hydroxycycloalkyl, wie beispielsweise 4-Hydroxycyclohexyl,

$C_1$-$C_5$-Alkoxy-$C_4$-$C_{12}$-Cycloalkyl, wie 4-Methoxycyclohexyl, 4-tert.-Butoxy-cyclohexyl,

$C_3$-$C_9$-Trialkylsilyl-$C_4$-$C_{12}$-Cycloalkyl, wie beispielsweise 4-Trimethylsilylcyclohexyl,

$C_4$-$C_{12}$-Cycloalkenyl, wie beispielsweise Cyclopentenyl, Cyclohexenyl, Cycloheptenyl,

$C_4$-$C_{20}$-Alkylcycloalkyl, wie beispielsweise, 3,3-Dimethyl-cyclopentyl, 3,3,5-Trimethylcyclopentyl, 4-Methylcyclohexyl, 3-Methyl-Cyclohexyl, 3,3-Dimethylcyclohexyl, 3-Ethyl-cyclohexyl, 3,3,5-Trimethylcyclohexyl, 4-Isopropyl-cyclohexyl, 3-Ethyl-5-methyl-cyclohexyl, 4-tert.-Butyl-cyclohexyl, 4(2-Methyl-but-2-y)cyclohexyl, 4(2,4,4-Trimethyl-pent-2-yl)cyclohexyl,

$C_4$-$C_{20}$-Alkyl-hydroxycycloalkyl, wie beispielsweise 4-Hydroxy-3,6-dimethyl-cyclohexyl, 4-Hydroxy-3,3-dimethyl-cyclohexyl, 4-Hydroxy-3,3,5-trimethylcyclohexyl,

$C_4$-$C_{20}$-Cycloalkyl-alkyl, wie beispielsweise Cyclohexylmethyl, Cyclohexylethyl, Cyclohexylpropyl, $C_4$-$C_{20}$-Alkylcycloalkenyl, wie beispielsweise 4-Isopropyl-cyclohexenyl, 4-tert.-Butyl-cyclohexenyl, $C_9$-$C_{11}$ Bicycloalkyl, wie beispielsweise [4.3.0] Bicyclononyl, Decalyl, $C_9$-$C_{11}$-Hydroxybicycloalkyl, wie beispielsweise 6-Hydroxy-2-decalyl, 7-Hydroxy-2-decalyl,

$C_{10}$-$C_{15}$-Alkyl-bicycloalkyl, wie beispielsweise 9-Methyl-2-decalyl, 5,9-Dimethyl-2-decalyl, 5,5,9-Trimethyl-2-decalyl,

$C_{10}$-$C_{15}$-Alkyl-hydroxybicycloalkyl, wie beispielsweise 6-Hydroxy-9-methyl-2-decalyl, 6-Hydroxy-5,9-dimethyl-2-decalyl, 6-Hydroxy-5,5,9-Trimethyl-2-decalyl, $C_8$-$C_{10}$-Alkoxy-cycloalkyl z.B. 1,4-Dioxa-spiro[4,5]-decan-8-yl,

5- bis 7-gliedriges Heterocycloalkyl mit 1 oder 2 Heteroatomen aus der Gruppe Sauerstoff oder Schwefel, wie beispielsweise Tetrahydropyranyl, Tetrahydrothiopyranyl, Dioxanyl,

5- bis 7-gliedriges Heterocycloalkylmethyl mit 1 oder 2 Heteroatomen aus der Gruppe Sauerstoff oder Schwefel, wie beispielsweise Tetrahydropyranylmethyl, Dioxanylmethyl,

$C_1$-$C_8$-Alkyl-substituiertes 5 bis 7-gliedriges Heterocycloalkylmethyl mit 1 oder 2 Heteroatomen aus der Gruppe Sauerstoff oder Schwefel, wie beispielsweise 3,5-Diethyl-dioxan-2-yl-methyl, 3,6-Diethyl-dioxan-2-yl-methyl, 3,5-Dimethyl-dioxan-2-yl-methyl,

und der Rest $R^2$ verzweigtes $C_3$-$C_{10}$-Alkyl oder verzweigtes $C_3$-$C_8$-Alkoxy, wie beispielsweise Isopropyl, sec. Butyl, Isobutyl, tert. Butyl, 2-Methyl-but-2-yl, 2,4,4-Trimethyl-pent-2-yl, i-Propyloxy, sec.-Butoxy, tert.-Butoxy,

$C_3$-$C_7$-Cycloalkyl, wie Cyclopentyl, Cyclohexyl, Cycloheptyl, $C_3$-$C_8$-Alkylthio, wie beispielsweise tert.-Butyl-thio, sec.-Butyl-thio, iso-Buthyl-thio, n-Butyl-thio bedeuten.

Die Verbindungen der Formel I können beispielsweise hergestellt werden durch N-Alkylierung von N-Alkylpiperizinen der Struktur II

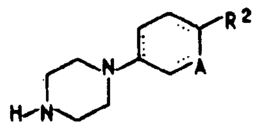

(II)

a) Umsetzung der Verbindung II mit einer Verbindung $R^1$-X unter basischen Bedingungen.

Als Reste X seien beispielsweise genannt, Chlor, Brom, Jod, die Methan-, Benzol-, p-Toluol-sulfonylgruppe oder die den oben genannten Anionen X entsprechenden Reste.

Die Reaktion wird beispielsweise bei 40-200°C, gegebenenfalls in Gegenwart eines inerten Lösungsmittels durchgeführt. Als Basen werden anorganische Basen bevorzugt, beispielsweise Kalium-, Natrium- und Lithiumhydroxid, Natriumhydrid, Kalium-und Natriumcarbonat. Geeignet sind auch organische Basen wie Triethylamin, Dicyclohexylamin und Diisopropylamin. Die Reaktion läßt sich auch mit einem Überschuß des Piperazinderivates II durchführen.

b) Umsetzung einer Carbonylverbindung III

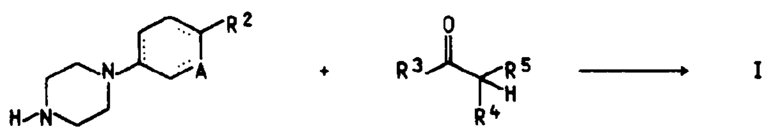

(II) (III)

wobei die Reste $R^3$ bis $R^5$ so definiert sind, daß der Rest IV

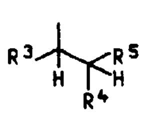

(IV)

in seiner Gesamtheit dem Rest $R^1$ entspricht, mit einem Piperazinderivat II unter gleichzeitiger oder anschließender Reduktion bzw. Hydrierung.

$b_1$) Bei der direkten Methode zur Herstellung der Verbindung I wird ein Gemisch aus II und III in Gegenwart eines Lösungsmittels, beispielsweise Methanol, Ethanol, Propanol, Isopropanol, die bis zu 25 % (Vol.) Wasser enthalten können, mit Natriumcyanborhydrid oder Natriumborhydrid, gegebenenfalls in Gegenwart eines Metallsalzes wie beispielsweise Zink(II)chlorid bei 0-100°C, vorzugsweise 20-80°C oder aber in Gegenwart eines Lösungsmittels wie beispielsweise Methanol, Ethanol, Tetrahydrofuran oder Toluol und eines Hydrierkatalysators wie z.B. Raney-Nickel, Platin-IV-oxid, $Ru_20_3$ oder Palladium auf Kohle im Autoklaven bei 100-150°C mit Wasserstoff bis zur Druckkonstanz umgesetzt.

$b_2$) Bei der Zweistufen-Reaktion wird aus den Verbindungen II und III ein Enamin unter wasserentziehenden Bedingungen hergestellt und anschließend mit einem Edelmetallkatalysator wie Raney-Nickel, Raney-Cobalt, $Pt0_2$ oder $Ru_20_3$, vorzugsweise Palladium auf Kohle und Wasserstoff reduziert.

Die als Ausgangsverbindungen verwendeten N-monosubstituierten Piperazine II lassen sich durch den Piperazin-Ring aufbauende Reaktionen (siehe z.B. PRATT, Y.T.: The Pyrazines and Piperazines, in: R.C. ELDERFIELD ((Hrsg.): Heterocyclic Compounds, Vol. 6, New York-London, 1957) oder durch Derivatisierung von Piperazinderivaten herstellen. Mögliche Synthesen sind in Schema 1 aufgezeigt.

a) So kann man ein entsprechend substituiertes Amin VII mit Diethanolamin unter saurer Katalyse (HCl, HBr, $H_2S0_4$) bei 200-250°C oder aber mit N,N-Bischlorethylamin in einem organischen Lösungsmittel wie beispielsweise Diethylenglykoldimethylether, Ethanol, Aceton, Aceton/Wasser, Dimethylformamid in Gegenwart einer Hilfsbase wie beispielsweise Kalium- oder Natriumcarbonat bei 60-250°C zu II umsetzen. Im Fall, daß es sich bei der Verbindung VII um ein Anilin handelt, kann aus dem erhaltenen N-Phenylpiperazin-Derivat II durch katalytische Hydrierung das N-Cyclohexylpiperazin-Derivat II' erhalten werden.

b) Die N-Cycloaliphatisch-substituierten Piperazine II' lassen sich außerdem über die reduktive Aminierung von N-Formyl-piperazin VIII mit einem entsprechend substituierten Cycloalkanon IX durch ein geeignetes Reduktionsmittel, wie beispielsweise Natriumcyanborhydrid/Zinkchlorid in Methanol bei 20-60°C und anschließende alkalische Verseifung des N-Formyl-N'-Cycloalkylpiperazinderivates X gewinnen.

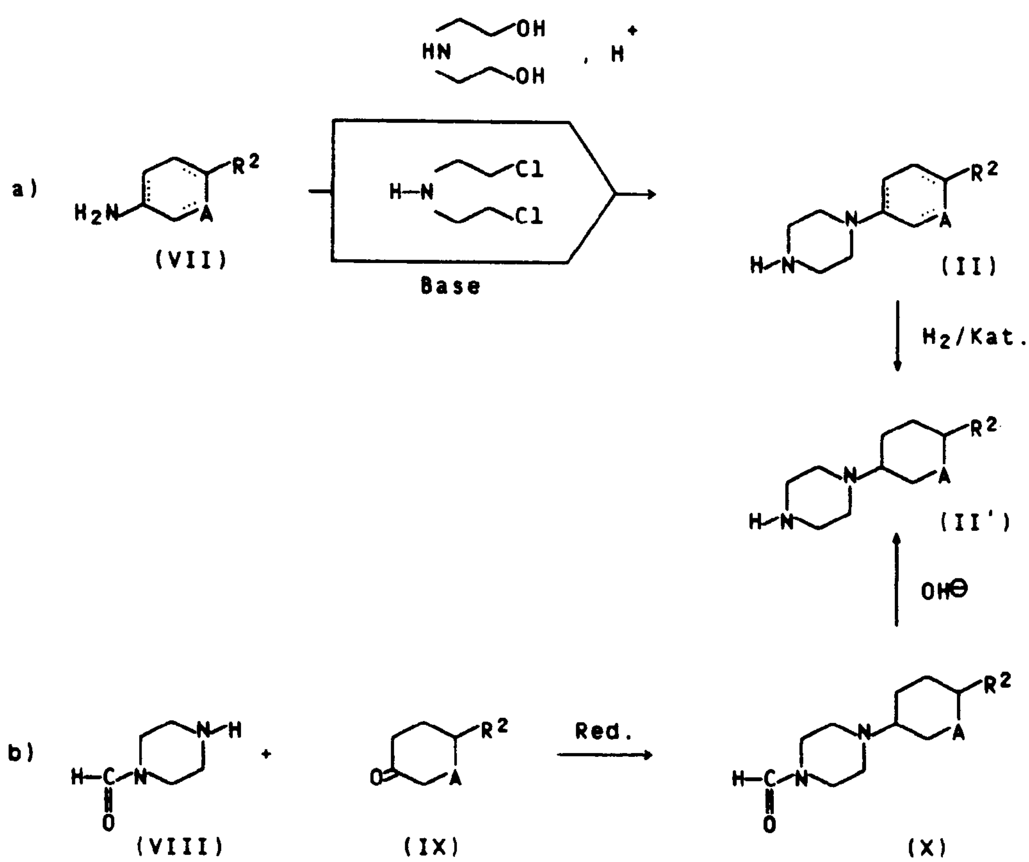


a) (VII) (II) (II')

b) (VIII) (IX) (X)

Die folgenden Beispiele erläutern die Herstellung der neuen Verbindungen:

Herstellungsbeispiele

Vorschrift 1 Herstellung der Ausgangsverbindung

N-(4-tert.-Butyl-phenyl)piperazin

149 g (1 mol) 4-tert.-Butylanilin, 178,5 g (1 mol) Bis(2-chlorethylamin)hydrochlorid und 138 g (1 mol) Kaliumcarbonat werden in 600 ml Diethylenglykoldimethylether 90 Std. zum Rückfluß erhitzt. Die Mischung wird mit 1 l Wasser und konz. Natronlauge stark alkalisch gestellt und 2 Std. gerührt. Die organische Phase wird mit 3 x 250 ml Methyl-tert.-butylether extrahiert, die vereinigten org. Phasen werden mit 2 x 250 ml Wasser gewaschen, über Natriumsulfat getrocknet und i.Vak. eingeengt. Der Rückstand wird i. Vak. fraktioniert: 155 g (71 % d.Th.), Sdp. 114-117°C/0,01 mbar, Schmp. 48-52°C.

Vorschrift 2 Herstellung der Ausgangsverbindung

N-(4-tert.-Butyl-cyclohexyl)piperazin

90 g (0,41 mol) N(4-tert.-Butylphenyl)piperazin werden in 1,6 l Dioxan an 5 g Ruthenium(IV)oxid bei 130°C und 150 bar 74 Std. hydriert. Die übliche Aufarbeitung ergibt einen weißen Feststoff.

Beispiel 1

N(3,3-Dimethyl-cyclohexyl)-N'-(4-tert.-butylcyclohexyl)piperazin (Verbindung Nr. 612)

5,0 g (22,3 mmol) N(4-tert.-Butycyclohexyl)piperazin, 5,4 g (44,6 mmol) 3,3-Dimethylcyclohexanon, 1,7 g (12,3 mmol) Zink(II)chlorid und 1,6 g (24,5 mmol) Natriumcyanborydrid werden 48 Std. bei 20°C gerührt. Die Mischung wird i.Vak. eingeengt, mit 1 N Natronlauge hydrolysiert, mit Methyl-tert.-butyl-ether extrahiert. Die übliche Aufarbeitung des org. Extraktes liefert 3,7 g (50 % d.Th.) Produkt in Form weißer Kristalle (Schmp. 90-92°C; IR-Daten siehe Tabelle 1b).

Beispiel 2

N(4-tert.-Butylphenyl)-N'(3,3-Dimethyl-butyl)piperazin (Verbindung Nr. 17)

5 g (22,9 mmol) N(4-tert.-Butylphenyl)piperazin, 8,3 g (68,7 mmol) Neohexylchlorid, 3,2 g (22,9 mmol) Kaliumcarbonat und 3,8 g (22,9 mmol) Kaliumiodid werden 16 Std. bei 140°C gerührt. Die Mischung wird in Dichlormethan und verdünnter Natronlauge aufgenommen. Die organische Phase wird gewaschen, über Natriumsulfat getrocknet, im Vakuum eingeengt und an Kieselgel mit Methyl-tert.-butylether/Hexan (1:1) chromatographiert: 1,5 g (22 % d. Th.) eines farblosen Öls (IR-Daten siehe Tabelle 1a).

Entsprechend Beispiel1 und 2 lassen sich die in Tabellen 1a/b aufgeführten erfindungsgemäßen Verbindungen herstellen.

Tabelle 1a

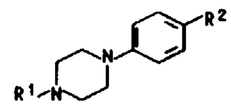

| Verb.-Nr. | $R^1$ | $R^2$ | Sdp.(°C/mbar) Schmp.(°C) | IR-Absorption ($cm^{-1}$) [Film] |
|---|---|---|---|---|
| 1 | n-Propyl | tert.-Butyl | | |
| 2 | Isopropyl | tert.-Butyl | | |
| 3 | n-Butyl | tert.-Butyl | 41-45 | 2957,2925,2871,2823,1517,1239,1227,819 |
| 4 | Isobutyl | tert.-Butyl | 35-36 | 2954,2926,2869,2816,1518,1454,1232,821 |
| 3 | sec.-Butyl | tert.-Butyl | | |
| 4 | tert.-Butyl | tert.-Butyl | | |
| 5 | n-Pentyl | tert.-Butyl | | |
| 6 | 3-Methyl-butyl | tert.-Butyl | 145/0,2 | 2955,2870,2817,1518,1466,1456,1363,1230 |
| 7 | 2,2-Dimethyl-propyl | tert.-Butyl | | |
| 8 | Pent-2-yl | tert.-Butyl | | |
| 9 | Pent-3-yl | tert.-Butyl | | |
| 10 | 1,2-Dimethyl-propyl | tert.-Butyl | | |
| 11 | n-Hexyl | tert.-Butyl | | |
| 12 | Hex-2-yl | tert.-Butyl | | |
| 13 | Hex-3-yl | tert.-Butyl | | |
| 14 | 1,2,2-Trimethyl-propyl | tert.-Butyl | | |
| 15 | 1,3-Dimethylbutyl | tert.-Butyl | | |
| 16 | 4-Methyl-pentyl | tert.-Butyl | | |
| 17 | 3,3-Dimethyl-butyl | tert.-Butyl | Öl | 2953,2866,2818,1611,1519,1456,1364, 1236,1131,820 |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 18 | n-Heptyl | tert.-Butyl | | |
| 19 | Hept-2-yl | tert.-Butyl | | |
| 20 | Hept-3-yl | tert.-Butyl | | |
| 21 | Hept-4-yl | tert.-Butyl | | |
| 22 | Diisopropyl-methyl | tert.-Butyl | | |
| 23 | 1,4-dimethyl-pentyl | tert.-Butyl | | |
| 24 | 4,4-Dimethyl-pentyl | tert.-Butyl | 65 | 2955,2903,2867,1517,1468,1365,1236,824 |
| 25 | 3,3-Dimethyl-2-hydroxybutyl | tert.-Butyl | 137-138 | 2964,2949,2833,1520,1365,1333,1232,819 |
| 26 | n-Octyl | tert.-Butyl | | |
| 27 | 2-Methyl-hept-3-yl | tert.-Butyl | | |
| 28 | 5-Methyl-hept-3-yl | tert.-Butyl | | |
| 29 | Oct-2-yl | tert.-Butyl | | |
| 30 | Oct-3-yl | tert.-Butyl | | |
| 31 | Oct-4-yl | tert.-Butyl | | |
| 32 | 5,5-Dimethyl-hexyl | tert.-Butyl | | |
| 33 | 2,4,4-Trimethyl-pentyl | tert.-Butyl | Öl | 2958,2899,2866,2828,2810,1516,1374,1237 |
| 34 | 6-Methyl-hept-2-yl | tert.-Butyl | | |
| 35 | n-Nonyl | tert.-Butyl | | |
| 36 | Non-2-yl | tert.-Butyl | | |
| 37 | Non-3-yl | tert.-Butyl | | |
| 38 | Non-4-yl | tert.-Butyl | | |
| 39 | Non-5-yl | tert.-Butyl | | |
| 40 | 2,5,5-Trimethyl-hexyl | tert.-Butyl | | |
| 41 | 2,6-Dimethyl-hept-4-yl | tert.-Butyl | | |
| 42 | 3,5,5-Trimethyl-hexyl | tert.-Butyl | Öl | 2955,2905,2868,2817,1519,1465,1457, 1364,1231 |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 43 | n-Decyl | tert.-Butyl | | |
| 44 | Dec-2-yl | tert.-Butyl | | |
| 45 | Dec-3-yl | tert.-Butyl | | |
| 46 | Dec-4-yl | tert.-Butyl | | |
| 47 | 2,3,5,5-Tetramethylhexyl | tert.-Butyl | | |
| 48 | n-Undecyl | tert.-Butyl | | |
| 49 | n-Dodecyl | tert.-Butyl | | |
| 50 | n-Tridecyl | tert.-Butyl | | |
| 51 | 1,5,9-Trimethyl-decyl | tert.-Butyl | | |
| 52 | n-Tetradecyl | tert.-Butyl | | |
| 53 | 2-Hydroxyethyl | tert.-Butyl | | |
| 54 | 3-Hydroxypropyl | tert.-Butyl | | |
| 55 | 4-Hydroxy-butyl | tert.-Butyl | | |
| 56 | 1-Hydroxy-but-2-yl | tert.-Butyl | | |
| 57 | 2-Hydroxy-but-3-yl | tert.-Butyl | | |
| 58 | 3-Chlorpropyl | tert.-Butyl | | |
| 59 | 6-Chlorhexyl | tert.-Butyl | | |
| 60 | 3-Chlor-2-butenyl | tert.-Butyl | 61 | 2957,2869,2811,1517,1453,1364, 1231,1148,1004,824 |
| 61 | Trichlorethyl | tert.-Butyl | | |
| 62 | 5-Chlor-pent-1-yl | tert.-Butyl | | |
| 63 | 3-Chlor-but-2-yl | tert.-Butyl | | |
| 64 | 3,3-Dichlor-prop-2-yl | tert.-Butyl | | |
| 65 | 2-Methoxyethyl | tert.-Butyl | | |
| 66 | 2-Ethoxyethyl | tert.-Butyl | | |
| 67 | 2-tert.-Butoxyethyl | tert.-Butyl | | |
| 68 | 3-tert.-Butoxy-propyl | tert.-Butyl | | |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 69 | 6-Methoxyhexyl | tert.-Butyl | | |
| 70 | 3-Methoxy-prop-2-yl | tert.-Butyl | | |
| 71 | Trimethylsilylethyl | tert.-Butyl | | |
| 72 | 3-Trimethylsilyl-propyl | tert.-Butyl | | |
| 73 | 6-Trimethylsilyl-hexyl | tert.-Butyl | | |
| 74 | Allyl | tert.-Butyl | | |
| 75 | 3-Methyl-but-2-en-yl | tert.-Butyl | | |
| 76 | 1,5,9-Trimethyl-deca-4,8-dienyl | tert.-Butyl | | |
| 77 | Propargyl | tert.-Butyl | | |
| 78 | 4,4-Dimethyl-but-in-1-yl | tert.-Butyl | | |
| 79 | Cyclopentyl | tert.-Butyl | | |
| 80 | 3-Methyl-cyclopentyl | tert.-Butyl | | |
| 81 | 3,3-Dimethyl-cyclopentyl | tert.-Butyl | | |
| 82 | 3,3,5-Trimethyl-cyclopentyl | tert.-Butyl | | |
| 83 | Cyclohexyl | | Öl | 2967,2948,2924,2853,2828,1518,1231 813 |
| 84 | 3-Methyl-cyclohexyl | | | |
| 85 | 3,3-Dimethyl-cyclohexyl | tert.-Butyl | 175/0,2 | 2951,2931,2862,2811,1519,1450,1235 820 |
| 86 | 3-Ethyl-cyclohexyl | tert.-Butyl | | |
| 87 | 3-Ethyl-5-methyl-cyclohexyl | tert.-Butyl | 80-82 | 2960,2923,2864,1520,1461,1449,1236 1228 |
| 88 | 4-Methyl-cyclohexyl | tert.-Butyl | | |
| 89 | Cycloheptyl | tert.-Butyl | | |
| 90 | 4-Isopropyl-cyclohexyl (Isomerengemisch) | tert.-Butyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 91 | 3,3,5-Trimethyl-cyclohexyl | tert.-Butyl | Öl | 2949,2905,2865,2820,1518,1452,1232,823 |
| 92 | 3,3,5,5-Tetramethylcyclohexyl | tert.-Butyl | 133 | 2952,2922,2900,2822,2813,1519,1234,820 |
| 93 | 4-Hydroxy-cyclohexyl | tert.-Butyl | | |
| 94 | 4-tert.-Butoxy-cyclohexyl | tert.-Butyl | | |
| 95 | Cyclohex-2-en-yl | tert.-Butyl | | |
| 96 | 4-tert.-Butyl-cyclohexyl (Isomerengemisch) | tert.-Butyl | 128-30 | 2963,2939,2866,2813,1521,1446,1366, 1246,1232 |
| 97 | trans-4-tert.-Butyl-cyclohexyl | tert.-Butyl | | |
| 98 | 4(2-Methyl-but-2-yl)cyclohexyl (Isomerengemisch) | tert.-Butyl | | |
| 99 | trans-4(2-Methyl-but-2-yl)-cyclohexyl | tert.-Butyl | | |
| 100 | 4(2,4,4-Trimethyl-pent-2-yl) cyclohexyl (Isomerengemisch) | tert.-Butyl | | |
| 101 | trans-4(2,4,4-Trimethyl-pent-2-yl)cyclohexyl | tert.-Butyl | | |
| 102 | 4-Hydroxy-3-methyl-cyclohexyl | tert.-Butyl | | |
| 103 | 4-Hydroxy-3,6-dimethyl-cyclohexyl | tert.-Butyl | | |
| 104 | 4-Hydroxy-3,3-dimethyl-cyclohexyl | tert.-Butyl | | |
| 105 | 4-Hydroxy-3,3,5-trimethyl-cyclohexyl | tert.-Butyl | | |
| 106 | Cyclohexylmethyl | tert.-Butyl | | |
| 107 | Cyclohexylethyl | tert.-Butyl | 75-77 | 2946,2920,2851,2818,1517,1447,1234,830 |
| 108 | 4-tert.-Butyl-cyclohex-3-en-yl | tert.-Butyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 109 | 4-tert.-Butyl-cyclohex-2-en-yl | tert.-Butyl | | |
| 110 | 1-Decalyl (cis/trans-Gemisch) | tert.-Butyl | | |
| 111 | 2-Decalyl (cis/trans-Gemisch) | tert.-Butyl | 80 | 2961,2922,2854,2825,1612,1518,1447,1364 1234,8177 |
| 112 | trans-2-Decalyl (eq/ax.-substituiert) | tert.-Butyl | | |
| 113 | eq.-trans-2-Decalyl | tert.-Butyl | | |
| 114 | 6-Hydroxy-2-decalyl | tert.-Butyl | | |
| 115 | 7-Hydroxy-2-decalyl | tert.-Butyl | | |
| 116 | 2-Decalylmethyl | tert.-Butyl | | |
| 117 | 9-Methyl-trans-2-decalyl | tert.-Butyl | | |
| 118 | 5,9-Dimethyl-trans-2-decalyl | tert.-Butyl | | |
| 119 | 5,5,9-Trimethyl-trans-2-decalyl | tert.-Butyl | | |
| 120 | 6-Hydroxy-9-methyl-2-decalyl | tert.-Butyl | | |
| 121 | 6-Hydroxy-5,9-dimethyl-2-decalyl | tert.-Butyl | | |
| 122 | 6-Hydroxy-5,5,9-trimethyl-2-decalyl | tert.-Butyl | | |
| 123 | Tetrahydropyran-4-yl | tert.-Butyl | | |
| 124 | Tetrahydrothiopyran-4-yl | tert.-Butyl | | |
| 125 | Dioxan-2-yl-methyl | tert.-Butyl | | |
| 126 | Tetrahydropyran-2-yl-methyl | tert.-Butyl | | |
| 127 | Tetrahydropyran-3-yl-methyl | tert.-Butyl | | |
| 128 | 3,5-Dimethyl-dioxan-2-yl-methyl | tert.-Butyl | | |
| 129 | 3,5-Diethyl-dioxan-2-yl-methyl | tert.-Butyl | | |
| 130 | 3,6-Diethyl-dioxan-2-yl-methyl | tert.-Butyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 131 | 1,4-Dioxa-spiro[4,5]decan-8-yl 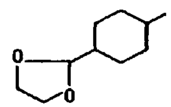 | tert.-Butyl | Öl | 2958,2882,1519,1250,1234,1148,1129 |
| 132 | 4-Trimethylsilyl-cyclohexyl | tert.-Butyl | | |
| 133 | n-Propyl | 1,1-Dimethyl-propyl | | |
| 134 | Isopropyl | 1,1-Dimethyl-propyl | | |
| 135 | n-Butyl | 1,1-Dimethyl-propyl | | |
| 136 | Isobutyl | 1,1-Dimethyl-propyl | | |
| 137 | sec.-Butyl | 1,1-Dimethyl-propyl | | |
| 138 | tert.-Butyl | 1,1-Dimethyl-propyl | | |
| 139 | n-Pentyl | 1,1-Dimethyl-propyl | | |
| 140 | 3-Methyl-butyl | 1,1-Dimethyl-propyl | | |
| 141 | 2,2-Dimethyl-propyl | 1,1-Dimethyl-propyl | | |
| 142 | Pent-2-yl | 1,1-Dimethyl-propyl | | |
| 143 | Pent-3-yl | 1,1-Dimethyl-propyl | | |
| 144 | 1,2-Dimethyl-propyl | 1,1-Dimethyl-propyl | | |
| 145 | n-Hexyl | 1,1-Dimethyl-propyl | | |
| 146 | Hex-2-yl | 1,1-Dimethyl-propyl | | |
| 147 | Hex-3-yl | 1,1-Dimethyl-propyl | | |
| 148 | 1,2,2-Trimethyl-propyl | 1,1-Dimethyl-propyl | | |
| 149 | 1,3-Dimethylbutyl | 1,1-Dimethyl-propyl | | 2959,2876,2818,1518,1465,1450,1364 1231 |
| 150 | 4-Methyl-pentyl | 1,1-Dimethyl-propyl | | |
| 151 | 3,3-Dimethyl-butyl | 1,1-Dimethyl-propyl | Harz | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 152 | n-Heptyl | 1,1-Dimethyl-propyl | | |
| 153 | Hept-2-yl | 1,1-Dimethyl-propyl | | |
| 154 | Hept-3-yl | 1,1-Dimethyl-propyl | | |
| 155 | Hept-4-yl | 1,1-Dimethyl-propyl | | |
| 156 | Diisopropyl-methyl | 1,1-Dimethyl-propyl | | |
| 157 | 1,4-dimethyl-pentyl | 1,1-Dimethyl-propyl | | |
| 158 | 4,4-Dimethyl-pentyl | 1,1-Dimethyl-propyl | Öl | 2955,2877,2817,1518,2475,1466,1364, 1247,1229 |
| 159 | 3,3-Dimethyl-2-hydroxy-butyl | 1,1-Dimethyl-propyl | | |
| 160 | n-Octyl | 1,1-Dimethyl-propyl | | |
| 161 | 2-Methyl-hept-3-yl | 1,1-Dimethyl-propyl | | |
| 162 | 5-Methyl-hept-3-yl | 1,1-Dimethyl-propyl | | |
| 163 | Oct-2-yl | 1,1-Dimethyl-propyl | | |
| 164 | Oct-3-yl | 1,1-Dimethyl-propyl | | |
| 165 | Oct-4-yl | 1,1-Dimethyl-propyl | | |
| 166 | 5,5-Dimethyl-hexyl | 1,1-Dimethyl-propyl | | |
| 167 | 2,4,4-Trimethyl-pentyl | 1,1-Dimethyl-propyl | Öl | 2959,2914,2877,2816,1518,1464,1455, 1229 |
| 168 | 6-Methyl-hept-2-yl | 1,1-Dimethyl-propyl | | |
| 169 | n-Nonyl | 1,1-Dimethyl-propyl | | |
| 170 | Non-2-yl | 1,1-Dimethyl-propyl | | |
| 171 | Non-3-yl | 1,1-Dimethyl-propyl | | |
| 172 | Non-4-yl | 1,1-Dimethyl-propyl | | |
| 173 | Non-5-yl | 1,1-Dimethyl-propyl | | |
| 174 | 2,5,5-Trimethyl-hexyl | 1,1-Dimethyl-propyl | | |
| 175 | 2,6-Dimethyl-hept-4-yl | 1,1-Dimethyl-propyl | | |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 176 | 3,5,5-Trimethyl-hexyl | 1,1-Dimethyl-propyl | | |
| 177 | n-Decyl | 1,1-Dimethyl-propyl | | |
| 178 | Dec-2-yl | 1,1-Dimethyl-propyl | | |
| 179 | Dec-3-yl | 1,1-Dimethyl-propyl | | |
| 180 | Dec-4-yl | 1,1-Dimethyl-propyl | | |
| 181 | 2,3,5,5-Tetramethylhexyl | 1,1-Dimethyl-propyl | | |
| 182 | n-Undecyl | 1,1-Dimethyl-propyl | | |
| 183 | n-Dodecyl | 1,1-Dimethyl-propyl | | |
| 184 | n-Tridecyl | 1,1-Dimethyl-propyl | | |
| 185 | 1,5,9-Trimethyl-decyl | 1,1-Dimethyl-propyl | | |
| 186 | n-Tetradecyl | 1,1-Dimethyl-propyl | | |
| 187 | 2-Hydroxyethyl | 1,1-Dimethyl-propyl | | |
| 188 | 3-Hydroxyethyl | 1,1-Dimethyl-propyl | | |
| 189 | 4-Hydroxy-butyl | 1,1-Dimethyl-propyl | | |
| 190 | 1-Hydroxy-but-2-yl | 1,1-Dimethyl-propyl | | |
| 191 | 2-Hydroxy-but-3-yl | 1,1-Dimethyl-propyl | | |
| 192 | 3-Chlorpropyl | 1,1-Dimethyl-propyl | | |
| 193 | 6-Chlorhexyl | 1,1-Dimethyl-propyl | | |
| 194 | 3-Chlor-2-butenyl | 1,1-Dimethyl-propyl | | |
| 195 | Trichlorethyl | 1,1-Dimethyl-propyl | | |
| 196 | 5-Chlor-pent-2-yl | 1,1-Dimethyl-propyl | | |
| 197 | 3-Chlor-but-2-yl | 1,1-Dimethyl-propyl | | |
| 198 | 3,3-Dichlor-prop-2-yl | 1,1-Dimethyl-propyl | | |
| 199 | 2-Methoxyethyl | 1,1-Dimethyl-propyl | | |
| 200 | 2-Ethoxyethyl | 1,1-Dimethyl-propyl | | |
| 201 | 2-tert.-Butoxyethyl | 1,1-Dimethyl-propyl | | |
| 202 | 3-tert.-Butoxy-ethyl | 1,1-Dimethyl-propyl | | |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 203 | 6-Methoxyhexyl | 1,1-Dimethyl-propyl | | |
| 204 | 3-Methoxy-prop-2-yl | 1,1-Dimethyl-propyl | | |
| 205 | Trimethylsilylethyl | 1,1-Dimethyl-propyl | | |
| 206 | 3-Trimethylsilyl-propyl | 1,1-Dimethyl-propyl | | |
| 207 | 6-Trimethylsilyl-hexyl | 1,1-Dimethyl-propyl | | |
| 208 | Allyl | 1,1-Dimethyl-propyl | | |
| 209 | 3-Methyl-but-2-en-yl | 1,1-Dimethyl-propyl | | |
| 210 | 1,5,9-Trimethyl-deca-4,8-dienyl | 1,1-Dimethyl-propyl | | |
| 211 | Propargyl | 1,1-Dimethyl-propyl | | |
| 212 | 4,4-Dimethyl-but-in-1-yl | 1,1-Dimethyl-propyl | | |
| 213 | Cyclopentyl | 1,1-Dimethyl-propyl | | |
| 214 | 3-Methyl-cyclopentyl | 1,1-Dimethyl-propyl | | |
| 215 | 3,3-Dimethyl-cyclopentyl | 1,1-Dimethyl-propyl | | |
| 216 | 3,3,5-Trimethyl-cyclopentyl | 1,1-Dimethyl-propyl | | |
| 217 | Cyclohexyl | 1,1-Dimethyl-propyl | 48 | 2966,2926,2855,2829,1519,1451,1248, 1228,813 |
| 218 | 3-Methyl-cyclohexyl | 1,1-Dimethyl-propyl | | |
| 219 | 3,3-Dimethyl-cyclohexyl | 1,1-Dimethyl-propyl | Öl | 2951,2930,2863,2811,1519,1463,1951, 1364,1235,819 |
| 220 | 3,3,5-Trimethyl-cyclohexyl | 1,1-Dimethyl-propyl | | |
| 221 | 3,3,5,5-Tetramethyl-cyclohexyl | 1,1-Dimethyl-propyl | | |
| 222 | 3-Ethyl-Cyclohexyl | 1,1-Dimethyl-propyl | | |
| 223 | 3-Ethyl-5-methyl-cyclohexyl | 1,1-Dimethyl-propyl | | |
| 224 | 4-Methyl-cyclohexyl | 1,1-Dimethyl-propyl | | |
| 225 | 4-Isopropyl-cyclohexyl | 1,1-Dimethyl-propyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 226 | Cycloheptyl | 1,1-Dimethyl-propyl | | |
| 227 | 4-Hydroxy-cyclohexyl | 1,1-Dimethyl-propyl | | |
| 228 | 4-tert.-Butoxy-cyclohexyl | 1,1-Dimethyl-propyl | | |
| 229 | 4-Trimetylsolyl-cyclohexyl (cis/trans-Gemisch) | 1,1-Dimethyl-propyl | | |
| 230 | Cyclohex-2-en-yl | 1,1-Dimethyl-propyl | | |
| 231 | 4-tert.-Butyl-cyclohexyl (Isomerengemisch) | 1,1-Dimethyl-propyl | 115 | 2964,2940,2865,2818,1520,1447,1366, 1248,1228,813 |
| 232 | trans-4-tert.-Butyl-cyclohexyl | 1,1-Dimethyl-propyl | | |
| 233 | 4(2-Methyl-but-2-yl)cyclohexyl (Isomerengemisch) | 1,1-Dimethyl-propyl | | |
| 234 | trans-4(2-Methyl-but-2-yl)-cyclohexyl | 1,1-Dimethyl-propyl | | |
| 235 | 4(2,4,4-Trimethyl-pent-2-yl)-cyclohexyl (Isomerengemisch) | 1,1-Dimethyl-propyl | | |
| 236 | trans-4(2,4,4-Trimethyl-pent-2-yl)cyclohexyl | 1,1-Dimethyl-propyl | | |
| 237 | 4-Hydroxy-3-methyl-cyclohexyl | 1,1-Dimethyl-propyl | | |
| 238 | 4-ydroxy-3,6-dimethyl-cyclohexyl | 1,1-Dimethyl-propyl | | |
| 239 | 4-Hydroxy-3,3-dimethyl-cyclo hexyl | 1,1-Dimethyl-propyl | | |
| 240 | 4-Hydroxy-3,3,5-trimethyl-cyclohexyl | 1,1-Dimethyl-propyl | | |
| 241 | Cyclohexylmethyl | 1,1-Dimethyl-propyl | | |
| 242 | Cyclohexylethyl | 1,1-Dimethyl-propyl | | |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 243 | 4-tert.-Butyl-cyclohex-3-en-yl | 1,1-Dimethyl-propyl | | |
| 244 | 4-tert.-Butyl-cyclohex-2-en-yl | 1,1-Diemthyl-propyl | | |
| 245 | 1-Decalyl (cis/trans-Gemisch) | 1,1-Dimethyl-propyl | | |
| 246 | 2-Decalyl (cis/trans-Gemisch) | 1,1-Dimethyl-propyl | | |
| 247 | trans-2-Decalyl (eq/ax.-substituiert) | 1,1-Dimethyl-propyl | | |
| 248 | eq.-trans-2-Decalyl | 1,1-Dimethyl-propyl | | |
| 249 | 6-Hydroxy-2-decalyl | 1,1-Dimethyl-propyl | | |
| 250 | 7-Hydroxy-2-decalyl | 1,1-Dimethyl-propyl | | |
| 251 | 2-Decalylmethyl | 1,1-Dimethyl-propyl | | |
| 252 | 9-Methyl-trans-2-decalyl | 1,1-Dimethyl-propyl | | |
| 253 | 5,9-Dimethyl-trans-2-decalyl | 1,1-Dimethyl-propyl | | |
| 254 | 5,5,9-Trimethyl-trans-2-decalyl | 1,1-Dimethyl-propyl | | |
| 255 | 6-Hydroxy-9-methyl-2-decalyl | 1,1-Dimethyl-propyl | | |
| 256 | 6-Hydroxy-5,9-dimethyl-2-decalyl | 1,1-Dimethyl-propyl | | |
| 257 | 6-Hydroxy-5,5,9-trimethyl-2-decalyl | 1,1-Dimethyl-propyl | | |
| 258 | Tetrahydropyran-4-yl | 1,1-Dimethyl-propyl | | |
| 259 | Tetrahydrothiopyran-4-yl | 1,1-Dimethyl-propyl | | |
| 260 | Dioxan-2-yl-methyl | 1,1-Dimethyl-propyl | | |
| 261 | Tetrahydropyran-2-yl-methyl | 1,1-Dimethyl-propyl | | |
| 262 | Tetrahydropyran-3-yl-methyl | 1,1-Dimethyl-propyl | | |
| 263 | 3,5-Dimethyl-dioxan-2-yl-methyl | 1,1-Dimethyl-propyl | | |
| 264 | 3,5-Diethyl-dioxan-2-yl-methyl | 1,1-Dimethyl-propyl | | |
| 265 | 3,6-Diethyl-dioxan-2-yl-methyl | 1,1-Dimethyl-propyl | | |
| 266 | | | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 267 | 1,4-Dioxa-spiro[4,5-decan]-8-yl | 1,1-Dimethyl-propyl | | |
| 268 | n-Propyl | sec.-Butyl | | |
| 269 | Isopropyl | sec.-Butyl | | |
| 270 | n-Butyl | sec.-Butyl | | |
| 271 | Isobutyl | sec.-Butyl | | |
| 272 | sec.-Butyl | sec.-Butyl | | |
| 273 | tert.-Butyl | sec.-Butyl | | |
| 274 | n-Pentyl | sec.-Butyl | | |
| 275 | 3-Methyl-butyl | sec.-Butyl | | |
| 276 | 2,2-Dimethyl-propyl | sec.-Butyl | | |
| 277 | Pent-2-yl | sec.-Butyl | | |
| 278 | Pent-3-yl | sec.-Butyl | | |
| 279 | 1,2-Dimethyl-propyl | sec.-Butyl | | |
| 280 | n-Hexyl | sec.-Butyl | | |
| 281 | Hex-2-yl | sec.-Butyl | | |
| 282 | Hex-3-yl | sec.-Butyl | | |
| 283 | 1,2,2-Trimethyl-propyl | sec.-Butyl | | |
| 284 | 1,3-Dimethyl-butyl | sec.-Butyl | | |
| 285 | 4-Methyl-pentyl | sec.-Butyl | | |
| 286 | 3,3-Dimethyl-butyl | sec.-Butyl | 50 | 2959,2871,2820,1612,1516,1456,1234, 822 |
| 287 | n-Heptyl | sec.-Butyl | | |
| 288 | Hept-2-yl | sec.-Butyl | | |
| 289 | Hept-3-yl | sec.-Butyl | | |
| 290 | Hept-4-yl | sec.-Butyl | | |
| 291 | Diisopropyl-methyl | sec.-Butyl | | |
| 292 | 1,4-Dimethyl-pentyl | sec.-Butyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 293 | 4,4-Dimethyl-pentyl | sec.-Butyl | Öl | 2955,2872,2816,1516,1454,1377,1346,1246,1232,823 |
| 294 | 3,3-Dimethyl-2-hydroxy-butyl | sec.-Butyl | | |
| 295 | n-Octyl | sec.-Butyl | | |
| 296 | 2-Methyl-hept-3-yl | sec.-Butyl | | |
| 297 | 5-Methyl-hept-3-yl | sec.-Butyl | | |
| 298 | Oct-2-yl | sec.-Butyl | | |
| 299 | Oct-3-yl | sec.-Butyl | | |
| 300 | Oct-4-yl | sec.-Butyl | | |
| 301 | 5,5-Dimethyl-hexyl | sec.-Butyl | | |
| 302 | 2,4,4-Trimethyl-pentyl | sec.-Butyl | | |
| 303 | 6-Methyl-hept-2-yl | sec.-Butyl | | |
| 304 | n-Nonyl | sec.-Butyl | | |
| 305 | Non-2-yl | sec.-Butyl | | |
| 306 | Non-3-yl | sec.-Butyl | | |
| 307 | Non-4-yl | sec.-Butyl | | |
| 308 | Non-5-yl | sec.-Butyl | | |
| 309 | 2,5,5-Trimethyl | sec.-Butyl | | |
| 310 | 2,6-Dimethyl-hept-4-yl | sec.-Butyl | | |
| 311 | 3,5,5-Trimethyl-hexyl | sec.-Butyl | | |
| 312 | n-Decyl | sec.-Butyl | | |
| 313 | Dec-2-yl | sec.-Butyl | | |
| 314 | Dec-3-yl | sec.-Butyl | | |
| 315 | Dec-4-yl | sec.-Butyl | | |
| 316 | 2,3,5,5-Tetramethyl-hexyl | sec.-Butyl | | |
| 317 | n-Undecyl | sec.-Butyl | | |
| 318 | n-Dodecyl | sec.-Butyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 319 | n-Tridecyl | sec.-Butyl | | |
| 320 | 1,5,9-Trimethyl-decyl | sec.-Butyl | | |
| 321 | n-Tetradecyl | sec.-Butyl | | |
| 322 | 2-Hydroxyethyl | sec.-Butyl | | |
| 323 | 3-Hydroxypropyl | sec.-Butyl | | |
| 324 | 4-Hydroxy-butyl | sec.-Butyl | | |
| 325 | 1-Hydroxy-but-2-yl | sec.-Butyl | | |
| 326 | 2-Hydroxy-but-3-yl | sec.-Butyl | | |
| 327 | 3-Chlorpropyl | sec.-Butyl | | |
| 328 | 6-Chlorhexyl | sec.-Butyl | | |
| 329 | 5-Chlor-pent-2-yl | sec.-Butyl | | |
| 330 | 3-Chlor-but-2-yl | sec.-Butyl | | |
| 331 | 3,3-Dichlor-prop-2-yl | sec.-Butyl | | |
| 332 | 2-Methoxyethyl | sec.-Butyl | | |
| 333 | 2-Ethoxyethyl | sec.-Butyl | | |
| 334 | 2-tert.-Butoxyethyl | sec.-Butyl | | |
| 335 | 3-tert.-Butoxy-ethyl | sec.-Butyl | | |
| 336 | 6-Methoxyhexyl | sec.-Butyl | | |
| 337 | 3-Methoxy-prop-2-yl | sec.-Butyl | | |
| 338 | Trimethylsilylethyl | sec.-Butyl | | |
| 339 | 3-Trimethylsilyl-propyl | sec.-Butyl | | |
| 340 | 6-Trimethylsilyl-hexyl | sec.-Butyl | | |
| 341 | Allyl | sec.-Butyl | | |
| 342 | 3-Methyl-but-2-en-yl | sec.-Butyl | | |
| 343 | 1,5,9-Trimethyl-deca-4,8-dienyl | sec.-Butyl | | |
| 344 | Progargyl | sec.-Butyl | | |
| 345 | 4,4-Dimethyl-but-in-1-yl | sec.-Butyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 346 | Cyclopentyl | sec.-Butyl | | |
| 347 | 3-Methyl-cyclopentyl | sec.-Butyl | | |
| 348 | 3,3-Dimethyl-cyclopentyl | sec.-Butyl | | |
| 349 | 3,3,5-Trimethyl-cyclopentyl | sec.-Butyl | | |
| 350 | Cyclohexyl | sec.-Butyl | | |
| 351 | 3-Methyl-cyclohexyl | sec.-Butyl | | |
| 352 | 3,3-Dimethyl-cyclohexyl | sec.-Butyl | 55 | 2953,2928,2873,2819,1517,1452,1378,1234 |
| 356 | 3,3,5-Trimethyl-cyclohexyl | sec.-Butyl | | |
| 354 | 3,3,5,5-Tetramethyl-cyclohexyl | sec.-Butyl | | |
| 355 | 3-Ethyl-Cyclohexyl | sec.-Butyl | | |
| 356 | 3-Ethyl-5-methyl-cyclohexyl | sec.-Butyl | | |
| 357 | 4-Methyl-cyclohexyl | sec.-Butyl | | |
| 358 | 4-Isopropyl-cyclohexyl | sec.-Butyl | | |
| 359 | Cycloheptyl | sec.-Butyl | | |
| 360 | 4-Hydroxy-cyclohexyl | sec.-Butyl | | |
| 361 | 4-tert.-Butoxy-cyclohexyl | sec.-Butyl | | |
| 362 | 4-Trimethylsilyl-cyclohexyl (cis/trans-Gemisch) | sec.-Butyl | | |
| 363 | Cyclohex-2-en-yl | sec.-Butyl | | |
| 364 | 4-tert.-Butyl-cyclohexyl (Isomerengemisch) | sec.-Butyl | | |
| 365 | trans-4-tert.-Butyl-cyclohexyl | sec.-Butyl | | |
| 366 | 4(2-Methyl-but-2-yl)cyclohexyl (Isomerengemisch) | sec.-Butyl | | |
| 367 | trans-4(2-Methyl-but-2-yl)-cyclohexyl | sec.-Butyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 368 | 4(2,4,4-Trimethyl-pent-2-yl)-cyclohexyl (Isomerengemisch) | sec.-Butyl | | |
| 369 | trans-4(2,4,4-Trimethyl-pent-2-yl)cyclohexyl | sec.-Butyl | | |
| 370 | 4-Hydroxy-3-methyl-cyclohexyl | sec.-Butyl | | |
| 371 | 4-hydroxy-3,6-dimethyl-cyclohexyl | sec.-Butyl | | |
| 372 | 4-Hydroxy-3,3-dimethyl-cyclohexyl | sec.-Butyl | | |
| 373 | 4-Hydroxy-3,3,5-trimethyl-cyclohexyl | sec.-Butyl | | |
| 374 | Cyclohexylmethyl | sec.-Butyl | | |
| 375 | Cyclohexylethyl | sec.-Butyl | | |
| 376 | 4-tert.-Butyl-cyclohex-3-en-yl | sec.-Butyl | | |
| 377 | 4-tert.-Butyl-cyclohex-2-en-yl | sec.-Butyl | | |
| 378 | 1-Decalyl (cis/trans-Gemisch) | sec.-Butyl | | |
| 379 | 2-Decalyl (cis/trans-Gemisch) | sec.-Butyl | | |
| 380 | trans-2-Decalyl (eq/ax.-substituiert) | sec.-Butyl | | |
| 381 | eq.-trans-2-Decalyl | sec.-Butyl | | |
| 382 | 6-Hydroxy-2-Decalyl | sec.-Butyl | | |
| 383 | 7-Hydroxy-2-Decalyl | sec.-Butyl | | |
| 384 | 2-Decalylmethyl | sec.-Butyl | | |
| 385 | 9-Methyl-trans-2-decalyl | sec.-Butyl | | |
| 386 | 5,9-Dimethyl-trans-2-decalyl | sec.-Butyl | | |
| 387 | 5,5,9-Trimethyl-trans-2-decalyl | sec.-Butyl | | |
| 388 | 6-Hydroxy-9-methyl-2-decalyl | sec.-Butyl | | |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 389 | 6-Hydroxy-5,9-dimethyl-2-decalyl | sec.-Butyl | | |
| 390 | 6-Hydroxy-5,5,9-trimethyl-2-decalyl | sec.-Butyl | | |
| 391 | Tetrahydropyran-4-yl | sec.-Butyl | | |
| 392 | Tetrahydrothiopyran-4-yl | sec.-Butyl | | |
| 393 | Dioxan-2-yl-methyl | sec.-Butyl | | |
| 394 | Tetrahydropyran-2-yl-methyl | sec.-Butyl | | |
| 395 | Tetrahydropyran-3-yl-methyl | sec.-Butyl | | |
| 396 | 3,5-Dimethyl-dioxan-2-yl-methyl | sec.-Butyl | | |
| 397 | 3,5-Diethyl-dioxan-2-yl-methyl | sec.-Butyl | | |
| 398 | 3,6-Diethyl-dioxan-2-yl-methyl | sec.-Butyl | | |
| 399 | 1,4-Dioxa-spiro[4,5-decan]-8-yl | sec.-Butyl | | |
| 400 | n-Propyl | Cyclohexyl | | |
| 401 | Isopropyl | Cyclohexyl | | |
| 402 | n-Butyl | Cyclohexyl | | |
| 403 | Isobutyl | Cyclohexyl | | |
| 404 | sec.-Butyl | Cyclohexyl | | |
| 405 | tert.-Butyl | Cyclohexyl | | |
| 406 | n-Pentyl | Cyclohexyl | | |
| 407 | 3-Methyl-butyl | Cyclohexyl | | |
| 408 | 2,2-Dimethyl-propyl | Cyclohexyl | | |
| 409 | Pent-2-yl | Cyclohexyl | | |
| 410 | Pent-3-yl | Cyclohexyl | | |
| 411 | 1,2-Dimethyl-propyl | Cyclohexyl | | |
| 412 | n-Hexyl | Cyclohexyl | | |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 413 | Hex-2-yl | Cyclohexyl | | |
| 414 | Hex-3-yl | Cyclohexyl | | |
| 415 | 1,2,2-Trimethyl-propyl | Cyclohexyl | | |
| 416 | 1,3-Dimethyl-butyl | Cyclohexyl | | |
| 417 | 4-Methyl-pentyl | Cyclohexyl | | |
| 418 | 3,3-Dimethyl-butyl | Cyclohexyl | Öl | 2958,2924,2852,2819,1518,1235,1233,829 |
| 419 | n-Heptyl | Cyclohexyl | | |
| 420 | Hept-2-yl | Cyclohexyl | | |
| 421 | Hept-3-yl | Cyclohexyl | | |
| 422 | Hept-4-yl | Cyclohexyl | | |
| 423 | Diisopropyl-methyl | Cyclohexyl | | |
| 424 | 1,4-Dimethyl-pentyl | Cyclohexyl | | |
| 425 | 4,4-Dimethyl-pentyl | Cyclohexyl | Öl | 2953,2925,1517,1448,1364,1251,1241,814 |
| 426 | 3,3-Dimethyl-2-hydroxy-butyl | Cyclohexyl | | |
| 427 | n-Octyl | Cyclohexyl | | |
| 428 | 2-Methyl-hept-3-yl | Cyclohexyl | | |
| 429 | 5-Methyl-hept-3-yl | Cyclohexyl | | |
| 430 | 5,5-Dimethyl-hexyl | Cyclohexyl | | |
| 431 | 2,4,4-Trimethylpentyl | Cyclohexyl | 56 | 2956,2926,2845,1518,1447,1239,1229,814 |
| 432 | 6-Methyl-hept-2-yl | Cyclohexyl | | |
| 433 | n-Nonyl | Cyclohexyl | | |
| 434 | 2,5,5-Trimethyl-hexyl | Cyclohexyl | | |
| 435 | 2,6-Dimethyl-hept-4-yl | Cyclohexyl | | |
| 436 | 3,5,5-Trimethyl-hexyl | Cyclohexyl | | |
| 437 | n-Decyl | Cyclohexyl | | |
| 438 | 2,3,5,5-Tetramethyl-hexyl | Cyclohexyl | | |
| 439 | n-Undecyl | Cyclohexyl | | |

Tabelle 1a - Forts.

| Verb.-Nr. | $R^1$ | $R^2$ | Sdp.($^0$C/mbar) Schmp.($^0$C) | IR-Absorption (cm$^{-1}$) [Film] |
|---|---|---|---|---|
| 440 | n-Dodecyl | Cyclohexyl | | |
| 441 | n-Tridecyl | Cyclohexyl | | |
| 442 | 1,5,9-Trimethyl-decyl | Cyclohexyl | | |
| 443 | n-Tetradecyl | Cyclohexyl | | |
| 444 | 2-Hydroxyethyl | Cyclohexyl | | |
| 445 | 3-Hydroxyethyl | Cyclohesyl | | |
| 446 | 4-Hydroxy-butyl | Cyclohexyl | | |
| 447 | 1-Hydroxy-but-2-yl | Cyclohexyl | | |
| 448 | 2-Hydroxy-but-3-yl | Cyclohexyl | | |
| 449 | 3-Chlorpropyl | Cyclohexyl | | |
| 450 | 6-Chlorhexyl | Cyclohexyl | | |
| 451 | 5-Chlor-pent-2-yl | Cyclohexyl | | |
| 452 | 3-Chlor-but-2-yl | Cyclohexyl | | |
| 453 | 3,3-Dichlor-prop-2-yl | Cyclohexyl | | |
| 454 | 2-Methoxyethyl | Cyclohexyl | | |
| 455 | 2-Ethoxyethyl | Cyclohexyl | | |
| 456 | 2-tert.-Butoxyethyl | Cyclohexyl | | |
| 457 | 3-tert.-Butoxy-ethyl | Cyclohexyl | | |
| 458 | 6-Methoxyhexyl | Cyclohexyl | | |
| 459 | 3-Methoxy-prop-2-yl | Cyclohexyl | | |
| 460 | Trimethylsilylethyl | Cyclohexyl | | |
| 461 | 3-Trimethylsilyl-propyl | Cyclohexyl | | |
| 462 | 6-Trimethylsilyl-hexyl | Cyclohexyl | | |
| 463 | Allyl | Cyclohexyl | | |
| 464 | 3-Methyl-but-2-en-yl | Cyclohexyl | | |
| 465 | 1,5,9-Trimethyl-deca-4,8-dienyl | Cyclohexyl | | |
| 466 | Propargyl | Cyclohexyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 467 | 4,4-Dimethyl-but-in-1-yl | Cyclohexyl | | |
| 468 | Cyclopentyl | Cyclohexyl | | |
| 469 | 3-Methyl-cyclopentyl | Cyclohexyl | | |
| 470 | 3,3-Dimethyl-cyclopentyl | Cyclohexyl | | |
| 471 | 3,3,5-Trimethyl-cyclopentyl | Cyclohexyl | | |
| 472 | Cyclohexyl | Cyclohexyl | Harz | |
| 473 | 3-Methyl-cyclohexyl | Cyclohexyl | | |
| 474 | 3,3-Dimethyl-cyclohexyl | | Öl | 2926,2847,1611,1516,1448,1240,1226, 1134,815 |
| 475 | 3,3,5-Trimethyl-cyclohexyl | Cyclohexyl | | |
| 476 | 3,3,5,5-Trimethyl-cyclohexyl | Cyclohexyl | | |
| 477 | 3-Ethyl-Cyclohexyl | Cyclohexyl | | |
| 478 | 3-Ethyl-5-methyl-cyclohexyl | Cyclohexyl | | |
| 479 | 4-Methyl-cyclohexyl | Cyclohexyl | | |
| 480 | 4-Isopropyl-cyclohexyl | Cyclohexyl | | |
| 481 | Cycloheptyl | Cyclohexyl | | |
| 482 | 4-Hydroxy-cyclohexyl | Cyclohexyl | | |
| 483 | 4-tert.-Butoxy-cyclohexyl | Cyclohexyl | | |
| 484 | 4-Trimethylsilylcyclohexyl (cis/trans-Gemisch) | Cyclohexyl | | |
| 485 | Cyclohex-2-en-yl | Cyclohexyl | | |
| 486 | 4-tert.-Butyl-cyclohexyl (Isomerengemisch) | Cyclohexyl | | |
| 487 | trans-4-tert.-Butyl-cyclohexyl | Cyclohexyl | | |
| 488 | 4(2-Methyl-but-2-yl)cyclohexyl (Isomerengemisch) | Cyclohexyl | | |

Tabelle 1a - Forts.

| Verb.-Nr. | $R^1$ | $R^2$ | Sdp. (°C/mbar) Schmp. (°C) | IR-Absorption ($cm^{-1}$) [Film] |
|---|---|---|---|---|
| 489 | trans-4(2-Methyl-but-2-yl)-cyclohexyl | Cyclohexyl | | |
| 490 | 4(2,4,4-Trimethyl-pent-2-yl)-cyclohexyl (Isomerengemisch) | Cyclohexyl | | |
| 491 | trans-4(2,4,4-Trimethyl-pent-2-yl)cyclohexyl | Cyclohexyl | | |
| 492 | 4-Hydroxy-3-methyl-cyclohexyl | Cyclohexyl | | |
| 493 | 4-Hydroxy-3,6-dimethyl-cyclohexyl | Cyclohexyl | | |
| 494 | 4-Hydroxy-3,3-dimethyl-cyclohexyl | Cyclohexyl | | |
| 495 | 4-Hydroxy-3,3,5-trimethyl-cyclohexyl | Cyclohexyl | | |
| 496 | Cyclohexylmethyl | Cyclohexyl | | |
| 497 | Cyclohexylethyl | Cyclohexyl | | |
| 498 | 4-tert.-Butyl-cyclohex-3-en-yl | Cyclohexyl | | |
| 499 | 4-tert.-Butyl-cyclohex-2-en-yl | Cyclohexyl | | |
| 500 | 1-Decalyl (cis/trans-Gemisch) | Cyclohexyl | | |
| 501 | 2-Decalyl (cis/trans-Gemisch) | Cyclohexyl | | |
| 502 | trans-2-Decalyl (eq/ax.-substituiert) | Cyclohexyl | | |
| 503 | eq.-trans-2-Decalyl | Cyclohexyl | | |
| 504 | 6-Hydroxy-2-Decalyl | Cyclohexyl | | |
| 505 | 7-Hydroxy-2-Decalyl | Cyclohexyl | | |
| 506 | 2-Decalylmethyl | Cyclohexyl | | |
| 507 | 9-Methyl-trans-2-decalyl | Cyclohexyl | | |
| 508 | 5,9-Dimethyl-trans-2-decalyl | Cyclohexyl | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 509 | 5,5,9-Trimethyl-trans-2-decalyl | Cyclohexyl | | |
| 510 | 6-Hydroxy-9-methyl-2-decalyl | Cyclohexyl | | |
| 511 | 6-Hydroxy-5,9-dimethyl-2-decalyl | Cyclohexyl | | |
| 512 | 6-Hydroxy-5,5,9-trimethyl-2-decalyl | Cyclohexyl | | |
| 513 | Tetrahydropyran-4-yl | Cyclohexyl | | |
| 514 | Tetrahydrothiopyran-4-yl | Cyclohexyl | | |
| 515 | Dioxan-2-yl-methyl | Cyclohexyl | | |
| 516 | Tetrahydropyran-2-yl-methyl | Cyclohexyl | | |
| 517 | Tetrahydropyran-2-yl-methyl | Cyclohexyl | | |
| 518 | 3,5-Dimethyl-dioxan-2-yl-methyl | Cyclohexyl | | |
| 519 | 3,5-Diethyl-dioxan-2-yl-methyl | Cyclohexyl | | |
| 520 | 3,6-Diethyl-dioxan-2-yl-methyl | Cyclohexyl | | |
| 521 | 3,3-Dimethyl-butyl | tert.-Butoxy | | |
| 522 | 4,4-Dimethyl-pentyl | tert.-Butoxy | | |
| 523 | 2,4,4-Trimethyl-pentyl | tert.-Butoxy | | |
| 524 | 3,3-Dimethyl-cyclopentyl | tert.-Butoxy | | |
| 525 | 3,3,5-Triemethylcyclopentyl | tert.-Butoxy | | |
| 526 | 3-Methyl-cyclohexyl | tert.-Butoxy | | |
| 527 | 3,3-Dimethyl-cyclohexyl | tert.-Butoxy | | |
| 528 | 3-Ethyl-cyclohexyl | tert.-Butoxy | | |
| 529 | 4-Hydroxy-butyl | tert.-Butoxy | | |
| 530 | 2-tert.-Butoxy-ethyl | tert.-Butoxy | | |
| 531 | 3-tert.-Butoxy-propyl | tert.-Butoxy | | |
| 532 | 4-Hydroxy-cyclohexyl | tert.-Butoxy | | |

Tabelle 1a - Forts.

| Verb.-Nr. | $R^1$ | $R^2$ | Sdp.(°C/mbar) Schmp.(°C) | IR-Absorption ($cm^{-1}$) [Film] |
|---|---|---|---|---|
| 533 | 4-Hydroxy-3-methyl-cyclohexyl | tert.-Butoxy | | |
| 534 | 4-Hydroxy-3,3-dimethyl-cyclohexyl | tert.-Butoxy | | |
| 535 | 3-Ethyl-4-hydroxy-cyclohexyl | tert.-Butoxy | | |

Tabelle 1b

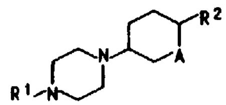

| Verb.-Nr. | A | $R^1$ | $R^2$ | Sdp($^0C$/mbar) Schmp.($^0C$) | IR-Absorp. ($cm^{-1}$) (Film) |
|---|---|---|---|---|---|
| 536 | - | 3,3-Dimethyl-butyl | tert.-Butyl | | |
| 537 | - | 4,4-Dimethyl-pentyl | tert.-Butyl | | |
| 538 | - | 3,3-Dimethyl-cyclopentyl | tert.-Butyl | | |
| 539 | - | 3,3,5-Trimethyl-cyclopentyl | tert.-Butyl | | |
| 540 | - | 3-Methyl-cyclohexyl | tert.-Butyl | | |
| 541 | - | 3,3-Dimethylcyclohexyl | tert.-Butyl | | |
| 542 | - | 3-Ethyl-cyclohexyl | tert.-Butyl | | |
| 543a | $CH_2$ | n-Propyl | tert.-Butyl (cis-trans-Isomereng.) | | |
| 543b | $CH_2$ | n-Propyl | tert.-Butyl (trans-Isomer) | | |
| 544a | $CH_2$ | Isopropyl | tert.-Butyl (cis-trans-Isomereng.) | | |
| 544b | $CH_2$ | Isopropyl | tert.-Butyl (trans-Isomer) | | |
| 545a | $CH_2$ | n-Butyl | tert.-Butyl (cis-trans-Isomereng.) | | |
| 545b | $CH_2$ | n-Butyl | tert.-Butyl (trans-Isomer) | | |
| 546a | $CH_2$ | Isobutyl | tert.-Butyl (cis-trans-Isomereng.) | | |
| 546b | $CH_2$ | Isobutyl | tert.-Butyl (trans-Isomer) | | |
| 547a | $CH_2$ | sec.-butyl | tert.-Butyl (cis-trans-Isomereng.) | | |
| 547b | $CH_2$ | sec.-butyl | tert.-Butyl (trans-Isomer) | | |
| 548a | $CH_2$ | tert.-Butyl | tert.-Butyl (cis-trans-Isomereng.) | | |
| 548b | $CH_2$ | tert.-Butyl | tert.-Butyl (trans-Isomer) | | |
| 549a | $CH_2$ | n-Pentyl | tert.-Butyl (cis-trans-Isomereng.) | | |
| 549b | $CH_2$ | n-Pentyl | tert.-Butyl (trans-Isomer) | | |
| 550a | $CH_2$ | 3-Methyl-butyl | tert.-Butyl (cis-trans-Isomereng.) | Öl | 2952,2869,2806,2767, 1469,1451,1366,1162 |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 550b | $CH_2$ | 3-Methyl-butyl | tert.-Butyl (trans-Isomer) | | |
| 551a | $CH_2$ | 2,2-Dimethyl-propyl | tert.-Butyl (cis-trans-Isomereng.) | | |
| 551b | $CH_2$ | 2,2-Dimethyl-propyl | tert.-Butyl (trans-Isomer) | | |
| 552a | $CH_2$ | Pent-2-yl | tert.-Butyl (cis-trans-Isomereng.) | | |
| 552b | $CH_2$ | Pent-2-yl | tert.-Butyl (trans-Isomer) | | |
| 553a | $CH_2$ | 1,2-Dimethyl-propyl | tert.-Butyl (cis-trans-Isomereng.) | | |
| 553b | $CH_2$ | 1,2-Dimethyl-propyl | tert.-Butyl (trans-Isomer) | | |
| 554a | $CH_2$ | n-Hexyl | tert.-Butyl (cis-trans-Isomereng.) | | |
| 554b | $CH_2$ | n-Hexyl | tert.-Butyl (trans-Isomer) | | |
| 555a | $CH_2$ | 1,2,2-Trimethyl-propyl | tert.-Butyl (cis-trans-Isomereng.) | | |
| 555b | $CH_2$ | 1,2,2-Trimethyl-propyl | tert.-Butyl (trans-Isomer) | | |
| 556a | $CH_2$ | 1,3-Dimethyl-butyl | tert.-Butyl (cis-trans-Isomereng.) | | |
| 556b | $CH_2$ | 1,3-Dimethyl-butyl | tert.-Butyl (trans-Isomer) | | |
| 557a | $CH_2$ | 4-Methyl-pentyl | tert.-Butyl (cis-trans-Isomereng.) | | |
| 557b | $CH_2$ | 4-Methyl-pentyl | tert.-Butyl (trans-Isomer) | | |
| 558a | $CH_2$ | 3,3-Dimethyl-butyl | tert.-Butyl (cis-trans-Isomereng.) | Öl | 2953,2867,2805,2765, 1471,1445,1364,1152 |
| 558b | $CH_2$ | 3,3-Dimethyl-butyl | tert.-Butyl (trans-Isomer) | | |
| 559a | $CH_2$ | n-Heptyl | tert.-Butyl (cis-trans-Isomereng.) | | |
| 559b | $CH_2$ | n-Heptyl | tert.-Butyl (trans-Isomer) | | |
| 560a | $CH_2$ | Hept-2-yl | tert.-Butyl (cis-trans-Isomereng.) | | |
| 560b | $CH_2$ | Hept-2-yl | tert.-Butyl (trans-Isomer) | | |
| 561a | $CH_2$ | Diisopropyl-methyl | tert.-Butyl (cis-trans-Isomereng.) | | |
| 561b | $CH_2$ | Diisopropyl-methyl | tert.-Butyl (trans-Isomer) | | |
| 562a | $CH_2$ | 1,4-Dimethyl-pentyl | tert.-Butyl (cis-trans-Isomereng.) | | |
| 562b | $CH_2$ | 1,4-Dimethyl-pentyl | tert.-Butyl (trans-Isomer) | | |
| 563a | $CH_2$ | 4,4-Dimethyl-pentyl | tert.-Butyl (cis-trans-Isomereng.) | | |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 563b | $CH_2$ | 4,4-Dimethyl-pentyl | tert.-Butyl (trans-Isomer) | | |
| 564a | $CH_2$ | 3,3-Dimethyl-2-hydroxybutyl | tert.-Butyl (cis-trans-Isomereng.) | | |
| 564b | $CH_2$ | 3,3-Dimethyl-2-hydroxybutyl | tert.-Butyl (trans-Isomer) | | |
| 565a | $CH_2$ | n-Octyl | tert.-Butyl (cis-trans-Isomereng.) | | |
| 565b | $CH_2$ | n-Octyl | tert.-Butyl (trans-Isomer) | | |
| 566a | $CH_2$ | 2-Methyl-hept-3-yl | tert.-Butyl (cis-trans-Isomereng.) | | |
| 566b | $CH_2$ | 2-Methyl-hept-3-yl | tert.-Butyl (trans-Isomer) | | |
| 567a | $CH_2$ | 5-Methyl-hept-3-yl | tert.-Butyl (cis-trans-Isomereng.) | | |
| 567b | $CH_2$ | 5-Methyl-hept-3-yl | tert.-Butyl (trans-Isomer) | | |
| 568a | $CH_2$ | 5,5-Dimethylhexyl | tert.-Butyl (cis-trans-Isomereng.) | | |
| 568b | $CH_2$ | 5,5-Dimethylhexyl | tert.-Butyl (trans-Isomer) | | |
| 569a | $CH_2$ | 2,4,4-Trimethyl-pentyl | tert.-Butyl (cis-trans-Isomereng.) | Öl | 2950,2867,2807,2767, 1476,1467,1457,1365 |
| 569b | $CH_2$ | 2,4,4-Trimethyl-pentyl | tert.-Butyl (trans-Isomer) | | |
| 570a | $CH_2$ | 6-Methyl-hept-2-yl | tert.-Butyl (cis-trans-Isomereng.) | | |
| 570b | $CH_2$ | 6-Methyl-hept-2-yl | tert.-Butyl (trans-Isomer) | | |
| 571a | $CH_2$ | n-Nonyl | tert.-Butyl (cis-trans-Isomereng.) | | |
| 571b | $CH_2$ | n-Nonyl | tert.-Butyl (trans-Isomer) | | |
| 572a | $CH_2$ | 2,5,5-Trimethyl-hexyl | tert.-Butyl (cis-trans-Isomereng.) | | |
| 572b | $CH_2$ | 2,5,5-Trimethyl-hexyl | tert.-Butyl (trans-Isomer) | | |
| 573a | $CH_2$ | 2,6-Dimethyl-hept-4-yl | tert.-Butyl (cis-trans-Isomereng.) | | |
| 573b | $CH_2$ | 2,6-Dimethyl-hept-4-yl | tert.-Butyl (trans-Isomer) | | |
| 574a | $CH_2$ | 3,5,5-Trimethyl-hexyl | tert.-Butyl (cis-trans-Isomereng.) | | |
| 574b | $CH_2$ | 3,5,5-Trimethyl-hexyl | tert.-Butyl (trans-Isomer) | | |
| 575a | $CH_2$ | n-Decyl | tert.-Butyl (cis-trans-Isomereng.) | | |
| 575b | $CH_2$ | n-Decyl | tert.-Butyl (trans-Isomer) | | |
| 576a | $CH_2$ | 2,3,5,5-Tetramethyl-hexyl | tert.-Butyl (cis-trans-Isomereng.) | | |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 576b | $CH_2$ | 2,3,5,5-Tetramethyl-hexyl | tert.-Butyl (trans-Isomer) | | |
| 577a | $CH_2$ | n-Undecyl | tert.-Butyl (cis-trans-Isomereng.) | | |
| 577b | $CH_2$ | n-Undecyl | tert.-Butyl (trans-Isomer) | | |
| 578a | $CH_2$ | n-Dodecyl | tert.-Butyl (cis-trans-Isomereng.) | | |
| 578b | $CH_2$ | n-Dodecyl | tert.-Butyl (trans-Isomer) | | |
| 579a | $CH_2$ | n-Tridecyl | tert.-Butyl (cis-trans-Isomereng.) | | |
| 579b | $CH_2$ | n-Tridecyl | tert.-Butyl (trans-Isomer) | | |
| 580a | $CH_2$ | 1,5,9-Trimethyl-decyl | tert.-Butyl (cis-trans-Isomereng.) | | |
| 580b | $CH_2$ | 1,5,9-Trimethyl-decyl | tert.-Butyl (trans-Isomer) | | |
| 581a | $CH_2$ | n-Tetradecyl | tert.-Butyl (cis-trans-Isomereng.) | | |
| 581b | $CH_2$ | n-Tetradecyl | tert.-Butyl (trans-Isomer) | | |
| 582a | $CH_2$ | 2-Hydroxyethyl | tert.-Butyl (cis-trans-Isomereng.) | | |
| 582b | $CH_2$ | 2-Hydroxyethyl | tert.-Butyl (trans-Isomer) | | |
| 583a | $CH_2$ | 3-Hydroxypropyl | tert.-Butyl (cis-trans-Isomereng.) | | |
| 583b | $CH_2$ | 3-Hydroxypropyl | tert.-Butyl (trans-Isomer) | | |
| 584a | $CH_2$ | 4-Hydroxy-butyl | tert.-Butyl (cis-trans-Isomereng.) | | |
| 584b | $CH_2$ | 4-Hydroxy-butyl | tert.-Butyl (trans-Isomer) | | |
| 585a | $CH_2$ | 1-Hydroxy-but-3-yl | tert.-Butyl (cis-trans-Isomereng.) | | |
| 585b | $CH_2$ | 1-Hydroxy-but-3-yl | tert.-Butyl (trans-Isomer) | | |
| 586a | $CH_2$ | 2-Hydroxy-but-3-yl | tert.-Butyl (cis-trans-Isomereng.) | | |
| 586b | $CH_2$ | 2-Hydroxy-but-3-yl | tert.-Butyl (trans-Isomer) | | |
| 587a | $CH_2$ | 3-Chlorpropyl | tert.-Butyl (cis-trans-Isomereng.) | | |
| 587b | $CH_2$ | 3-Chlorpropyl | tert.-Butyl (trans-Isomer) | | |
| 588a | $CH_2$ | 6-Chlorhexyl | tert.-Butyl (cis-trans-Isomereng.) | | |
| 588b | $CH_2$ | 6-Chlorhexyl | tert.-Butyl (trans-Isomer) | | |
| 589a | $CH_2$ | 5-Chlor-pent-2-yl | tert.-Butyl (cis-trans-Isomereng.) | | |
| 589b | $CH_2$ | 5-Chlor-pent-2-yl | tert.-Butyl (trans-Isomer) | | |

Tabelle 1b - Forts.

| Verb.-Nr. | A | $R^1$ | $R^2$ | Sdp($^0C$/mbar) Schmp.($^0C$) | IR-Absorp. ($cm^{-1}$) (Film) |
|---|---|---|---|---|---|
| 590a | $CH_2$ | 3-Chlor-but-2-yl | tert.-Butyl (cis-trans-Isomereng.) | | |
| 590b | $CH_2$ | 3-Chlor-but-2-yl | tert.-Butyl (trans-Isomer) | | |
| 591a | $CH_2$ | 3,3-Dichlor-prop-2-yl | tert.-Butyl (cis-trans-Isomereng.) | | |
| 591b | $CH_2$ | 3,3-Dichlor-prop-2-yl | tert.-Butyl (trans-Isomer) | | |
| 592a | $CH_2$ | 2-Methoxyethyl | tert.-Butyl (cis-trans-Isomereng.) | | |
| 592b | $CH_2$ | 2-Methoxyethyl | tert.-Butyl (trans-Isomer) | | |
| 593a | $CH_2$ | 2-Ethoxyethyl | tert.-Butyl (cis-trans-Isomereng.) | | |
| 593b | $CH_2$ | 2-Ethoxyethyl | tert.-Butyl (trans-Isomer) | | |
| 594a | $CH_2$ | 2-tert.-Butoxyethyl | tert.-Butyl (cis-trans-Isomereng.) | | |
| 594b | $CH_2$ | 2-tert.-Butoxyethyl | tert.-Butyl (trans-Isomer) | | |
| 595a | $CH_2$ | 3-tert.-Butoxy-ethyl | tert.-Butyl (cis-trans-Isomereng.) | | |
| 595b | $CH_2$ | 3-tert.-Butoxy-ethyl | tert.-Butyl (trans-Isomer) | | |
| 596a | $CH_2$ | 6-Methoxyhexyl | tert.-Butyl (cis-trans-Isomereng.) | | |
| 596b | $CH_2$ | 6-Methoxyhexyl | tert.-Butyl (trans-Isomer) | | |
| 597a | $CH_2$ | 3-Methoxy-prop-2-yl | tert.-Butyl (cis-trans-Isomereng.) | | |
| 597b | $CH_2$ | 3-Methoxy-prop-2-yl | tert.-Butyl (trans-Isomer) | | |
| 598a | $CH_2$ | Trimethylsilylethyl | tert.-Butyl (cis-trans-Isomereng.) | | |
| 598b | $CH_2$ | Trimethylsilylethyl | tert.-Butyl (trans-Isomer) | | |
| 599a | $CH_2$ | 3-Trimethylsilyl-propyl | tert.-Butyl (cis-trans-Isomereng.) | | |
| 599b | $CH_2$ | 3-Trimethylsilyl-propyl | tert.-Butyl (trans-Isomer) | | |
| 600a | $CH_2$ | 6-Trimethylsilyl-hexyl | tert.-Butyl (cis-trans-Isomereng.) | | |
| 600b | $CH_2$ | 6-Trimethylsilyl-hexyl | tert.-Butyl (trans-Isomer) | | |
| 601a | $CH_2$ | Allyl | tert.-Butyl (cis-trans-Isomereng.) | | |
| 601b | $CH_2$ | Allyl | tert.-Butyl (trans-Isomer) | | |
| 602a | $CH_2$ | 3-Methyl-but-2-en-yl | tert.-Butyl (cis-trans-Isomereng.) | | |
| 602b | $CH_2$ | 3-Methyl-but-2-en-yl | tert.-Butyl (trans-Isomer) | | |
| 603a | $CH_2$ | 1,5,9-Trimethyl-deca-4,8- | tert.-Butyl (cis-trans-Isomereng.) | | |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 603b | $CH_2$ | 1,5,9-Trimethyl-deca-4,8- | tert.-Butyl (trans-Isomer) | | |
| 604a | $CH_2$ | Propargyl | tert.-Butyl (cis-trans-Isomereng.) | | |
| 604b | $CH_2$ | Propargyl | tert.-Butyl (trans-Isomer) | | |
| 605a | $CH_2$ | 4,4-Dimethyl-but-in-1-yl | tert.-Butyl (cis-trans-Isomereng.) | | |
| 605b | $CH_2$ | 4,4-Dimethyl-but-in-1-yl | tert.-Butyl (trans-Isomer) | | |
| 606a | $CH_2$ | Cyclopentyl | tert.-Butyl (cis-trans-Isomereng.) | | |
| 606b | $CH_2$ | Cyclopentyl | tert.-Butyl (trans-Isomer) | | |
| 607a | $CH_2$ | 3-Methyl-cyclopentyl | tert.-Butyl (cis-trans-Isomereng.) | | |
| 607b | $CH_2$ | 3-Methyl-cyclopentyl | tert.-Butyl (trans-Isomer) | | |
| 608a | $CH_2$ | 3,3-Dimethyl-cyclopentyl | tert.-Butyl (cis-trans-Isomereng.) | | |
| 608b | $CH_2$ | 3,3-Dimethyl-cyclopentyl | tert.-Butyl (trans-Isomer) | | |
| 609a | $CH_2$ | 3,3,5-Trimethyl-cyclopentyl | tert.-Butyl (cis-trans-Isomereng.) | | |
| 609b | $CH_2$ | 3,3,5-Trimethyl-cyclopentyl | tert.-Butyl (trans-Isomer) | | |
| 610a | $CH_2$ | Cyclohexyl | tert.-Butyl (cis-trans-Isomereng.) | 75-78 | 2967,2940,2854,2804, 2758,14841363,1280, 1145 |
| 610b | $CH_2$ | Cyclohexyl | tert.-Butyl (trans-Isomer) | | |
| 611a | $CH_2$ | 3-Methyl-cyclohexyl | tert.-Butyl (cis-trans-Isomereng.) | | |
| 611b | $CH_2$ | 3-Methyl-cyclohexyl | tert.-Butyl (trans-Isomer) | | |
| 612a | $CH_2$ | 3,3-Dimethyl-cyclohexyl | tert.-Butyl (cis-trans-Isomereng.) | 90-92 | 2949,2864,2807,1471, 1447,1365,1339,1266, 1144 |
| 612b | $CH_2$ | 3,3-Dimethyl-cyclohexyl | tert.-Butyl (trans-Isomer) | | |
| 613a | $CH_2$ | 3,3,5-Trimethyl-cyclohexyl | tert.-Butyl (cis-trans-Isomereng.) | Öl | 2945,2864,2836,2804, 2764,1467,1452,1365 |
| 613b | $CH_2$ | 3,3,5-Trimethyl-cyclohexyl | tert.-Butyl (trans-Isomer) | | |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 614a | $CH_2$ | 3,3,5,5-Tetramethyl-cyclohexyl | tert.-Butyl (cis-trans-Isomereng.) | | |
| 614b | $CH_2$ | 3,3,5,5-Tetramethyl-cyclohexyl | tert.-Butyl (trans-Isomer) | | |
| 615a | $CH_2$ | 3-Ethyl-cyclohexyl | tert.-Butyl (cis-trans-Isomereng.) | | |
| 615b | $CH_2$ | 3-Ethyl-cyclohexyl | tert.-Butyl (trans-Isomer) | | |
| 616a | $CH_2$ | 3-Ethyl-5-methyl-cyclohexyl | tert.-Butyl (cis-trans-Isomereng.) | | |
| 616b | $CH_2$ | 3-Ethyl-5-methyl-cyclohexyl | tert.-Butyl (trans-Isomer) | | |
| 617a | $CH_2$ | 4-Methyl-cyclohexyl | tert.-Butyl (cis-trans-Isomereng.) | | |
| 617b | $CH_2$ | 4-Methyl-cyclohexyl | tert.-Butyl (trans-Isomer) | | |
| 618a | $CH_2$ | 4-Isopropyl-cyclohexyl | tert.-Butyl (cis-trans-Isomereng.) | | |
| 618b | $CH_2$ | 4-Isopropyl-cyclohexyl | tert.-Butyl (trans-Isomer) | | |
| 619a | $CH_2$ | Cycloheptyl | tert.-Butyl (cis-trans-Isomereng.) | | |
| 619b | $CH_2$ | Cycloheptyl | tert.-Butyl (trans-Isomer) | | |
| 620a | $CH_2$ | 4-Hydroxy-cyclohexyl | tert.-Butyl (cis-trans-Isomereng.) | | |
| 620b | $CH_2$ | 4-Hydroxy-cyclohexyl | tert.-Butyl (trans-Isomer) | | |
| 621a | $CH_2$ | 4-tert.-Butoxy-cyclohexyl | tert.-Butyl (cis-trans-Isomereng.) | | |
| 621b | $CH_2$ | 4-tert.-Butoxy-cyclohexyl | tert.-Butyl (trans-Isomer) | | |
| 622a | $CH_2$ | 4-Trimethylsolyl-cyclohexyl | tert.-Butyl (cis-trans-Isomereng.) | | |
| 622b | $CH_2$ | 4-Trimethylsolyl-cyclohexyl | tert.-Butyl (trans-Isomer) | | |
| 623a | $CH_2$ | Cyclohex-2-en-yl | tert.-Butyl (cis-trans-Isomereng.) | | |
| 623b | $CH_2$ | Cyclohex-2-en-yl | tert.-Butyl (trans-Isomer) | | |
| 624a | $CH_2$ | 4-tert.-Butyl-cyclohexyl | tert.-Butyl (cis-trans-Isomereng.) | 145-47 | 2965,2940,2862,2800, 2761,1454,1145,1366, 1278,1146 |
| 624b | $CH_2$ | 4-tert.-Butyl-cyclohexyl | tert.-Butyl (trans-Isomer) | | |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 625a | $CH_2$ | trans-4-tert.-Butyl-cyclohexyl | tert.-Butyl (cis-trans-Isomereng.) | | |
| 625b | $CH_2$ | trans-4-tert.-Butyl-cyclohexyl | tert.-Butyl (trans-Isomer) | | |
| 626a | $CH_2$ | 4(2-Methyl-but-2-yl)cyclohexyl | tert.-Butyl (cis-trans-Isomereng.) | | |
| 626b | $CH_2$ | 4(2-Methyl-but-2-yl)cyclohexyl | tert.-Butyl (trans-Isomer) | | |
| 627a | $CH_2$ | trans-4(2-Methyl-but-2-yl)-cyclohexyl | tert.-Butyl (cis-trans-Isomereng.) | | |
| 627b | $CH_2$ | trans-4(2-Methyl-but-2-yl)-cyclohexyl | tert.-Butyl (trans-Isomer) | | |
| 628a | $CH_2$ | 4(2,4,4-Trimethyl-pent-2-yl)-cyclohexyl | tert.-Butyl (cis-trans-Isomereng.) | | |
| 628b | $CH_2$ | 4(2,4,4-Trimethyl-pent-2-yl)-cyclohexyl | tert.-Butyl (trans-Isomer) | | |
| 629a | $CH_2$ | trans-4(2,4,4-Trimethyl-pent-2-yl)cyclohexyl | tert.-Butyl (cis-trans-Isomereng.) | | |
| 629b | $CH_2$ | trans-4(2,4,4-Trimethyl-pent-2-yl)cyclohexyl | tert.-Butyl (trans-Isomer) | | |
| 630a | $CH_2$ | 4-Hydroxy-3-methyl-cyclohexyl | tert.-Butyl (cis-trans-Isomereng.) | | |
| 630b | $CH_2$ | 4-Hydroxy-3-methyl-cyclohexyl | tert.-Butyl (trans-Isomer) | | |
| 631a | $CH_2$ | 4-Hydroxy-3,6-dimethyl-cyclohexyl | tert.-Butyl (cis-trans-Isomereng.) | | |

Tabelle 1b - Forts.

| Verb.-Nr. | A | $R^1$ | $R^2$ | Sdp($^0C$/mbar) Schmp.($^0C$) | IR-Absorp. ($cm^{-1}$) (Film) |
|---|---|---|---|---|---|
| 631b | $CH_2$ | 4-Hydroxy-3,6-dimethyl-cyclohexyl | tert.-Butyl (trans-Isomer) | | |
| 632a | $CH_2$ | 4-Hydroxy-3,3-dimethyl-cyclohexyl | tert.-Butyl (cis-trans-Isomereng.) | | |
| 632b | $CH_2$ | 4-Hydroxy-3,3-dimethyl-cyclohexyl | tert.-Butyl (trans-Isomer) | | |
| 633a | $CH_2$ | 4-Hydroxy-3,3,5-trimethyl-cyclohexyl | tert.-Butyl (cis-trans-Isomereng.) | | |
| 633b | $CH_2$ | 4-Hydroxy-3,3,5-trimethyl-cyclohexyl | tert.-Butyl (trans-Isomer) | | |
| 634a | $CH_2$ | Cyclohexylmethyl | tert.-Butyl (cis-trans-Isomereng.) | | |
| 634b | $CH_2$ | Cyclohexylmethyl | tert.-Butyl (trans-Isomer) | | |
| 635a | $CH_2$ | Cyclohexylethyl | tert.-Butyl (cis-trans-Isomereng.) | | |
| 635b | $CH_2$ | Cyclohexylethyl | tert.-Butyl (trans-Isomer) | | |
| 636a | $CH_2$ | 4-tert.-Butyl-cyclohex-3-en-yl | tert.-Butyl (cis-trans-Isomereng.) | | |
| 636b | $CH_2$ | 4-tert.-Butyl-cyclohex-3-en-yl | tert.-Butyl (tran-Isomer) | | |
| 637a | $CH_2$ | 4-tert.-Butyl-cyclohex-2-en-yl | tert.-Butyl (cis-trans-Isomereng.) | | |
| 637b | $CH_2$ | 4-tert.-Butyl-cyclohex-2-en-yl | tert.-Butyl (trans-Isomer) | | |
| 638a | $CH_2$ | 1-Decalyl | tert.-Butyl (cis-trans-Isomereng.) | | |
| 638b | $CH_2$ | 1-Decalyl | tert.-Butyl (trans-Isomer) | | |
| 639a | $CH_2$ | 2-Decalyl | tert.-Butyl (cis-trans-Isomereng.) | Harz | 2923,2858,1467,1449, 1365,1339,1158 |
| 639b | $CH_2$ | 2-Decalyl | tert.-Butyl (trans-Isomer) | | |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 640a | $CH_2$ | trans-2-Decalyl | tert.-Butyl (cis-trans-Isomereng.) | | |
| 640b | $CH_2$ | trans-2-Decalyl | tert.-Butyl (trans-Isomer) | | |
| 641a | $CH_2$ | eq.-trans-2-decalyl | tert.-Butyl (cis-trans-Isomereng.) | | |
| 641b | $CH_2$ | eq.-trans-2-decalyl | tert.-Butyl (trans-Isomer) | | |
| 642a | $CH_2$ | 6-Hydroxy-2-decalyl | tert.-Butyl (cis-trans-Isomereng.) | | |
| 642b | $CH_2$ | 6-Hydroxy-2-decalyl | tert.-Butyl (trans-Isomer) | | |
| 643a | $CH_2$ | 7-Hydroxy-2-decalyl | tert.-Butyl (cis-trans-Isomereng.) | | |
| 643b | $CH_2$ | 7-Hydroxy-2-decalyl | tert.-Butyl (trans-Isomer) | | |
| 644a | $CH_2$ | 2-Decalylmethyl | tert.-Butyl (cis-trans-Isomereng.) | | |
| 644b | $CH_2$ | 2-Decalylmethyl | tert.-Butyl (trans-Isomer) | | |
| 645a | $CH_2$ | 9-Methyl-trans-2-decalyl | tert.-Butyl (cis-trans-Isomereng.) | | |
| 645b | $CH_2$ | 9-Methyl-trans-2-decalyl | tert.-Butyl (trans-Isomer) | | |
| 646a | $CH_2$ | 5,9-Dimethyl-trans-2-decalyl | tert.-Butyl (cis-trans-Isomereng.) | | |
| 646b | $CH_2$ | 5,9-Dimethyl-trans-2-decalyl | tert.-Butyl (trans-Isomer) | | |
| 647a | $CH_2$ | 5,5,9-Trimethyl-trans-2-decalyl | tert.-Butyl (cis-trans-Isomereng.) | | |
| 647b | $CH_2$ | 5,5,9-Trimethyl-trans-2-decalyl | tert.-Butyl (trans-Isomer) | | |
| 648a | $CH_2$ | 6-Hydroxy-9-methyl-2-decalyl | tert.-Butyl (cis-trans-Isomereng.) | | |
| 648b | $CH_2$ | 6-Hydroxy-9-methyl-2-decalyl | | | |
| 649a | $CH_2$ | 6-Hydroxy-5,9-dimethyl-2-decalyl | tert.-Butyl (trans-Isomer) | | |

Tabelle 1b - Forts.

| Verb.-Nr. | A | $R^1$ | $R^2$ | Sdp(°C/mbar) Schmp.(°C) | IR-Absorp. ($cm^{-1}$) (Film) |
|---|---|---|---|---|---|
| 649b | $CH_2$ | 6-Hydroxy-5,9-dimethyl-2-decalyl | tert.-Butyl (cis-trans-Isomereng.) | | |
| 650a | $CH_2$ | 6-Hydroxy-5,5,9-trimethyl-2-decalyl | tert.-Butyl (trans-Isomer) | | |
| 650b | $CH_2$ | 6-Hydroxy-5,5,9-trimethyl-2-decalyl | tert.-Butyl (cis-trans-Isomereng.) | | |
| 651a | $CH_2$ | Tetrahydropyran-4-yl | tert.-Butyl (cis-trans-Isomereng.) | | |
| 651b | $CH_2$ | Tetrahydropyran-4-yl | tert.-Butyl (trans-Isomer) | | |
| 652a | $CH_2$ | Tetrahydrothiopyran-4-yl | tert.-Butyl (cis-trans-Isomereng.) | | |
| 652b | $CH_2$ | Tetrahydrothiopyran-4-yl | tert.-Butyl (trans-Isomer) | | |
| 653a | $CH_2$ | Dioxan-2-yl-methyl | tert.-Butyl (cis-trans-Isomereng.) | | |
| 653b | $CH_2$ | Dioxan-2-yl-methyl | tert.-Butyl (trans-Isomer) | | |
| 654a | $CH_2$ | Tetrahydropyran-2-yl-methyl | tert.-Butyl (cis-trans-Isomereng.) | | |
| 654b | $CH_2$ | Tetrahydropyran-2-yl-methyl | tert.-Butyl (trans-Isomer) | | |
| 655a | $CH_2$ | Tetrahydropyran-3-yl-methyl | tert.-Butyl (cis-trans-Isomereng.) | | |
| 655b | $CH_2$ | Tetrahydropyran-3-yl-methyl | tert.-Butyl (trans-Isomer) | | |
| 656a | $CH_2$ | 3,5-Dimethyl-dioxan-2-yl-methyl | tert.-Butyl (cis-trans-Isomereng.) | | |
| 656b | $CH_2$ | 3,5-Dimehyl-dioxan-2-yl-methyl | tert.-Butyl (trans-Isomer) | | |
| 657a | $CH_2$ | 3,5-Diethyl-dioxan-2-yl-methyl | tert.-Butyl (cis-trans-Isomereng.) | | |
| 657b | $CH_2$ | 3,5-Diethyl-dioxan-2-yl-methyl | tert.-Butyl (trans-Isomer) | | |
| 658a | $CH_2$ | 3,6-Diethyl-dioxan-2-yl-methyl | tert.-Butyl (cis-trans-Isomereng.) | | |

Tabelle 1b - Forts.

| Verb.-Nr. | A | $R^1$ | $R^2$ | Sdp($^0C$/mbar) Schmp.($^0C$) | IR-Absorp. ($cm^{-1}$) (Film) |
|---|---|---|---|---|---|
| 658b | $CH_2$ | 3,6-Diethyl-dioxan-2-yl-methyl | tert.-Butyl (trans-Isomer) | | |
| 659a | $CH_2$ | 1,4-Dioxa-spiro[4,5-decan]-8-yl | tert.-Butyl (cis-trans-Isomereng.) | | |
| 659b | $CH_2$ | 1,4-Dioxa-spiro[4,5-decan]-8-yl | tert.-Butyl (trans-Isomer) | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara Viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Einige der neuen Verbindungen haben sehr gute Wirksamkeit gegen humanpathogene Pilze, wie Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 17 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 85 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 558a werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 612a werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 17 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 85 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 558a werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 612a werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 17 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,

5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,

2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde N-Methyl-N'-3-(p-tert.butylphenyl)-2-methyl-propyl-piperazin (A) - bekannt aus DE 2 727 482 - benutzt.

Anwendungsbeispiel 1

Wirksamkeit gegen Gurkenmehltau

Blätter von in Töpfen gewachsenen Gurkenkeimlingen der Sorte "Chinesische Schlange" wurden im Zweiblattstadium mit einer Sporensuspension des Gurkenmehltaus besprüht. Nach etwa 20 Stunden wurden die Versuchspflanzen mit wäßriger Spritzbrühe, die 80 % (Gew.%) Wirkstoff und 20 % Emulgiermittel in der Trockensubstanz enthielt, bis zur Tropfnässe besprüht. Nach dem Antrocknen des Spritzbelages wurden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wurde das Ausmaß der Pilzentwicklung nach 21 Tagen ermittelt.

Das Ergebnis zeigt, daß die Wirkstoffe 17, 24, 25, 83, 87, 96, 107, 111, 217, 219, 231, 558a, 569a, 610a, 612a, 624a und 639a bei der Anwendung als 0,025 %ige Spritzbrühe eine bessere fungizide Wirkung (98 %) zeigen als der bekannte Wirkstoff A (80 %) bei 0,1 %iger Anwendung.

Anwendungsbeispiel 2

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Abtrocknen des Spritzbelages wurden die Versuchspflanzen im Gewächshaus bei Temperaturen zwischen 20 und 22 °C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 3, 4, 17, 24, 42, 60, 83, 85, 91, 96, 107, 111, 131, 151, 158, 167, 217, 219, 231, 293, 352, 418, 431, 474, 550a, 558a, 569a, 610a, 612a, 619a, 624a und 639a bei der Anwendung als 0,025 %ige ( Gew.%) Spritzbrühe eine bessere fungizide Wirkung zeigen (98 %) als der bekannte Vergleichswirkstoff A (60 %).

Anwendungsbeispiel 3

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer feuchten, wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 3, 60, 85, 107, 111, 151, 219, 474, 558a, 569a, 610a, 612a und 639a bei der Anwendung als 0,05 %ige Spritzbrühe eine bessere fungizide Wirkung (97 %) zeigen als der bekannte Vergleichswirkstoff A (60 %) bei 0,1 %iger Anwendung.

Anwendungsbeispiel 4

Wirksamkeit gegen Botrytis cinerea an Paprika

Paprikasämlinge der Sorte "Neusiedler Ideal Elite" wurden, nachdem sich 4 bis 5 Blätter gut entwickelt haben, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 85, 91, 111, 217, 231, 472, 474, 486 und 639a bei der Anwendung als 0,05 %ige Spritzbrühe eine bessere fungizide Wirkung (94 %) zeigen als der bekannte Wirkstoff A (10 %).

## Patentansprüche

1. N,N'-disubstitituierte Piperazine der allgemeinen Formel I

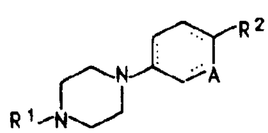

(I)

in der die Gruppe  ungesättigt (=) oder gesättigt (-) ist und die Substituenten $R^1$, $R^2$ und A die folgende Bedeutung haben:
A = -, -CH=, $-CH_2-$, $-CH_2-CH_2-$,
$R^1$ $C_2$-$C_{20}$-Alkyl, $C_3$-$C_{20}$-Alkenyl, $C_3$-$C_{20}$-Alkinyl, $C_4$-$C_{12}$-Cycloalkyl, $C_4$-$C_{12}$-Cycloalkenyl, $C_4$-$C_{20}$-Alkyl-cycloalkyl, $C_4$-$C_{20}$-Cycloalkenyl-alkyl, $C_4$-$C_{20}$-Alkyl-cycloalkenyl, $C_9$-$C_{11}$-Bicycloalkyl, $C_{10}$-$C_{15}$-Bicycloalkylalkyl, $C_{10}$-$C_{15}$-Alkyl-bicycloalkyl, wobei diese Reste gegebenenfalls durch Hydroxy, 1-3 Halogenatome, $C_1$-$C_5$-Alkoxy oder $C_3$-$C_9$-Trialkylsilyl substituiert sind,
$R^1$ ferner 5- bis 7-gliedriges Heterocycloalkyl mit 1 oder 2 Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl-substituiertes 5 bis 7-gliedriges Heterocycloalkylmethyl mit 1 oder 2 Heteroatomen aus der Gruppe Sauerstoff und/oder Schwefel,
$R^2$ verzweigtes $C_3$-$C_{10}$-Alkyl, verzweigtes $C_3$-$C_8$-Alkoxy, Trimethylsilyl, $C_3$-$C_7$-Cycloalkyl, $C_3$-$C_8$-Alkylthio, und deren pflanzenphysiologisch verträgliche Salze.

2. Verfahren zur Herstellung der Verbindungen gemäß Anspruch 1, dadurch gekennzeichnet, daß man
a) die Verbindung II

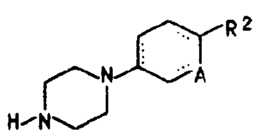

(II)

mit einer Verbindung der Formel $R^1$-X, in der der Substituent X einen in basischem Milieu abspaltbaren Rest bedeutet, unter basischen Bedingungen umsetzt, oder
b) die Verbindung II mit einer Carbonylverbindung III,

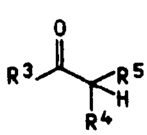

(III)

wobei die Reste $R^3$ bis $R^5$ so definiert sind, daß der Rest IV in seiner Gesamtheit dem Rest $R^1$

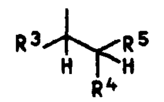

(IV)

entspricht,

$b_1$) unter reduzierenden Bedingungen reagieren läßt, oder

$b_2$) zu einem Enamin umsetzt und dieses katalytisch hydriert, oder mit Ameisensäure nach Leukart-Wallach reduziert.

3. Fungizid, enthaltend ein N,N'-disubstitituiertes Piperazin der allgemeinen Formel I

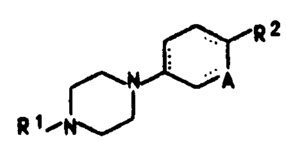

(I)

in der die Gruppe = ungesättigt (=) oder gesättigt (-) ist und die Substituenten $R^1$, $R^2$ und A die folgende Bedeutung haben:

A = -, -CH=, $-CH_2-$, $-CH_2-CH_2-$,

$R^1$ $C_2$-$C_{20}$-Alkyl, $C_3$-$C_{20}$-Alkenyl, $C_3$-$C_{20}$-Alkinyl, $C_4$-$C_{12}$-Cycloalkyl, $C_4$-$C_{12}$-Cycloalkenyl, $C_4$-$C_{20}$-Alkyl-cycloalkyl, $C_4$-$C_{20}$-Cycloalkenyl-alkyl, $C_4$-$C_{20}$-Alkyl-cycloalkenyl, $C_9$-$C_{11}$-Bicycloalkyl, $C_{10}$-$C_{15}$-Bicycloalkylalkyl, $C_{10}$-$C_{15}$-Alkyl-bicycloalkyl, wobei diese Reste gegebenenfalls durch Hydroxy, 1-3 Halogenatome, $C_1$-$C_5$-Alkoxy oder $C_3$-$C_9$-Trialkylsilyl substituiert sind,

$R^1$ ferner 5- bis 7-gliedriges Heterocycloalkyl mit 1 oder 2 Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl-substituiertes 5 bis 7-gliedriges Heterocycloalkylmethyl mit 1 oder 2 Heteroatomen aus der Gruppe Sauerstoff und/oder Schwefel,

$R^2$ verzweigtes $C_3$-$C_{10}$-Alkyl, verzweigtes $C_3$-$C_8$-Alkoxy, Trimethylsilyl, $C_3$-$C_7$-Cycloalkyl, $C_3$-$C_8$-Alkylthio, oder dessen pflanzenphysiologisch verträgliches Salz neben hierfür üblichen Trägerstoffen.

4. Verwendung der Verbindungen gemäß Anspruch 1 als Fungizide.

5. Verfahren zur Bekämpfung von Pilzen, dadurch gekennzeichnet, daß man die Pilze oder die vom Pilzbefall bedrohten Materialien, Pflanzen, Saatgüter oder Böden mit einem N,N'-disubstitituierten Piperazin der allgemeinen Formel I

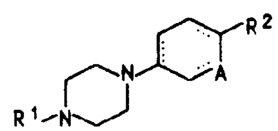

(I)

in der die Gruppe = ungesättigt (=) oder gesättigt (-) ist und die Substituenten $R^1$, $R^2$ und A die folgende Bedeutung haben:

A = -, -CH=, $-CH_2-$, $-CH_2-CH_2-$,

$R^1$ $C_2$-$C_{20}$-Alkyl, $C_3$-$C_{20}$-Alkenyl, $C_3$-$C_{20}$-Alkinyl, $C_4$-$C_{12}$-Cycloalkyl, $C_4$-$C_{12}$-Cycloalkenyl, $C_4$-$C_{20}$-Alkyl-cycloalkyl, $C_4$-$C_{20}$-Cycloalkenyl-alkyl, $C_4$-$C_{20}$-Alkyl-cycloalkenyl, $C_9$-$C_{11}$-Bicycloalkyl, $C_{10}$-$C_{15}$-Bicycloalkylalkyl, $C_{10}$-$C_{15}$-Alkyl-bicycloalkyl, wobei diese Reste gegebenenfalls durch Hydroxy, 1-3 Halogenatome, $C_1$-$C_5$-Alkoxy oder $C_3$-$C_9$ Trialkylsilyl substituiert sind,

$R^1$ ferner 5- bis 7-gliedriges Heterocycloalkyl mit 1 oder 2 Heteroatomen aus der Gruppe Sauerstoff und/oder Schwefel, 5 bis 7-gliedriges Heterocycloalkylmethyl mit 1 oder 2 Heteroatomen aus der Gruppe Sauerstoff und/oder Schwefel, $C_1$-$C_8$-Alkyl-substituiertes 5 bis 7-gliedriges Heterocycloalkylmethyl mit 1 oder 2 Heteroatomen

aus der Gruppe Sauerstoff und/oder Schwefel,
$R^2$ verzweigtes $C_3$-$C_{10}$-Alkyl, verzweigtes $C_3$-$C_8$-Alkoxy, Trimethylsilyl $C_3$-$C_7$-Cycloalkyl, $C_3$-$C_8$-Alkylthio, oder dessen pflanzenphysiologisch verträglichem Salz behandelt.

6. Verbindung gemäß Anspruch 1, dadurch gekennzeichnet, daß
die Gruppe $\underline{\cdots}$ ungesättigt (=) ist,
A die Gruppe -CH=
$R^1$ 3,3-Dimethylcyclohexyl und
$R^2$ tertiär Butyl bedeutet.

7. Verbindung gemäß Anspruch 1, dadurch gekennzeichnet, daß
die Gruppe $\underline{\cdots}$ gesättigt (-) ist,
A die Gruppe -$CH_2$-
$R^1$ 3,3-Dimethylcyclohexyl und
$R^2$ tertiär Butyl bedeutet.

8. Verbindung gemäß Anspruch 1, dadurch gekennzeichnet, daß
die Gruppe $\underline{\cdots}$ ungesättigt (=) ist,
A die Gruppe -CH=
$R^1$ 3,3-Dimehylbutyl und
$R^2$ tertiär Butyl bedeutet.

9. Verbindung gemäß Anspruch 1, dadurch gekennzeichnet, daß
die Gruppe $\underline{\cdots}$ gesättigt (-) ist,
A die Gruppe -$CH_2$-
$R^1$ 3,3-Dimethylbutyl und
$R^2$ tertiär Butyl bedeutet.

## Claims

1. An N,N'-disubstituted piperazine of the general formula I

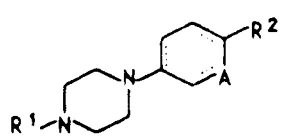

(I)

where the group $\underline{\cdots}$ is unsaturated ( = ) or saturated ( - ) and the substituents $R^1$, $R^2$ and A have the following meanings:
A is -, -CH=, -$CH_2$-, -$CH_2$-$CH_2$-,
$R^1$ is $C_2C_{20}$-alkyl, $C_3$-$C_{20}$-alkenyl, $C_3$-$C_{20}$-alkynyl, $C_4$-$C_{12}$-cycloalkyl, $C_4$-$C_{12}$-cycloalkenyl, $C_4$-$C_{20}$-alkylcycloalkyl, $C_4$-$C_{20}$-cycloalkenylalkyl, $C_4$-$C_{20}$-alkylcycloalkenyl, $C_9$-$C_{11}$-bicycloalkyl, $C_{10}$-$C_{15}$-bicycloalkylalkyl or $C_{10}$-$C_{15}$-alkylbicycloalkyl, these radicals being unsubstituted or substituted by hydroxy, 1 to 3 halogen atoms, $C_1$-$C_5$-alkoxy or $C_3$-$C_9$-trialkylsilyl,
$R^1$ is further 5- to 7-membered heterocycloalkyl with 1 or 2 heteroatoms selected from the group consisting of oxygen or sulfur, 5 - to 7 -mmbered heterocycloalkylmethyl with 1 or 2 heteroatoms selected from the group consisting of oxygen or sulfur, $C_1$-$C_8$-alkyl-substituted 5-to 7-membered heterocycloalkylmethyl with 1 or 2 heteroatoms selected from the group consisting of oxygen or sulfur, and
$R^2$ is branched $C_3$-$C_{10}$-alkyl, branched $C_3$-$C_8$-alkoxy, trimethylsilyl, $C_3$-$C_7$-cycloalkyl, $C_3$-$C_8$-alkylthio, and plant-physiologically tolerated salts thereof.

2. A process for the preparation of a compound as claimed in claim 1, wherein
a) a compound II

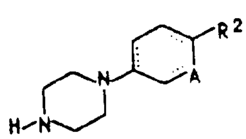

(II)

is reacted with a compound of the formula $R^1$-X, in which X is a radical which can be eliminated in a basic medium, under basic conditions, or

b) a compound II is reacted with a carbonyl compound III

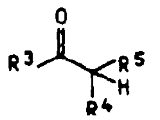 (III)

where $R^3$ to $R^5$ are defined so that the radical IV in its entirety corresponds to the radical $R^1$,

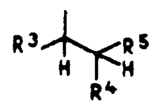 (IV)

$b_1$) under reducing conditions, or

$b_2$) to give an enamine and the latter is catalytically hydrogenated or reduced with formic acid by a Leukart-Wallach reaction.

3. A fungicide containing an N,N'-disubstituted piperazine of the general formula I

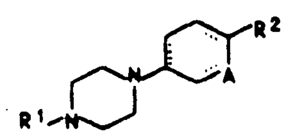 (I)

where the group $\underline{\cdots}$ is unsaturated (=) or saturated (-) and the substituents $R^1$, $R^2$ and A have the following meanings:

A is -, -CH=, $-CH_2-$, $-CH_2-CH_2-$,

$R^1$ is $C_2$-$C_{20}$-alkyl, $C_3$-$C_{20}$-alkenyl, $C_3$-$C_{20}$-alkynyl, $C_4$-$C_{12}$-cycloalkyl, $C_4$-$C_{12}$-cycloalkenyl, $C_4$-$C_{20}$-alkylcycloalkyl, $C_4$-$C_{20}$-cycloalkenylalkyl, $C_4$-$C_{20}$-alkylcycloalkenyl, $C_9$-$C_{11}$-bicycloalkyl, $C_{10}$-$C_{15}$-bicycloalkylalkyl or $C_{10}$-$C_{15}$-alkyl-bicycloalkyl, these radicals being unsubstituted or substituted by hydroxy, 1 to 3 halogen atoms, $C_1$-$C_5$-alkoxy or $C_3$-$C_9$-trialkylsilyl,

$R^1$ is further 5- to 7-membered heterocycloalkyl with 1 or 2 heteroatoms selected from the group consisting of oxygen or sulfur, 5- to 7 -membered heterocycloalkylmethyl with 1 or 2 heteroatoms selected from the group consisting of oxygen or sulfur, $C_1$-$C_8$-alkyl-substituted 5- to 7-membered heterocycloalkylmethyl with 1 or 2 heteroatoms selected from the group consisting of oxygen or sulfur, and

$R^2$ is branched $C_3$-$C_{10}$-alkyl, branched $C_3$-$C_8$-alkoxy, trimethylsilyl, $C_3$-$C_7$-cycloalkyl, $C_3$-$C_8$-alkylthio, or a plant-physiologically tolerated salt thereof, and conventional carriers.

4. Use of a compound as claimed in claim 1 as a fungicide.

5. A process for controlling fungi, wherein the fungi, or the materials, plants, seed or soil threatened by fungus attack are treated with an N,N'-disubstituted piperazine of the general formula I

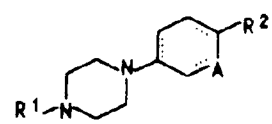 (I)

where the group $\underline{\cdots}$ is unsaturated (=) or saturated (-) and the substituents $R^1$, $R^2$ and A have the following meanings:

A is -, -CH=, $-CH_2-$, $-CH_2-CH_2-$,

$R^1$ is $C_2$-$C_{20}$-alkyl, $C_3$-$C_{20}$-alkenyl, $C_3$-$C_{20}$-alkynyl, $C_4$-$C_{12}$-cycloalkyl, $C_4$-$C_{12}$-cycloalkenyl, $C_4$-$C_{20}$-alkylcycloalkyl, $C_4$-$C_{20}$-cycloalkenylalkyl, $C_4$-$C_{20}$-alkylcycloalkenyl, $C_9$-$C_{11}$-bicycloalkyl, $C_{10}$-$C_{15}$-bicycloalkylalkyl or $C_{10}$-

$C_{15}$-alkyl-bicycloalkyl, these radicals being unsubstituted or substituted by hydroxy, 1 to 3 halogen atoms, $C_1$-$C_5$-alkoxy or $C_3$-$C_9$-trialkylsilyl,
$R^1$ is further 5- to 7-membered heterocycloalkyl with 1 or 2 heteroatoms selected from the group consisting of oxygen or sulfur, 5- to 7 -membered heterocycloalkylmethyl with 1 or 2 heteroatoms selected from the group consisting of oxygen or sulfur, $C_1$-$C_8$-alkyl-substituted 5- to 7-membered heterocycloalkylmethyl with 1 or 2 heteroatoms selected from the group consisting of oxygen or sulfur, and
$R^2$ is branched $C_3$-$C_{10}$-alkyl, branched $C_3$-$C_8$-alkoxy, trimethylsilyl, $C_3$-$C_7$-cycloalkyl, $C_3$-$C_8$-alkylthio, or a plant-physiologically tolerated salt thereof.

6. A compound as claimed in claim 1, wherein

the group $\underline{\cdots}$ is unsaturated (=),
A is the group -CH=,
$R^1$ is 3,3-dimethylcyclohexyl, and
$R^2$ is tert-butyl.

7. A compound as claimed in claim 1, wherein

the group $\underline{\cdots}$ is saturated (-),
A is the group $-CH_2-$,
$R^1$ is 3,3-dimethylcyclohexyl, and
$R^2$ is tert-butyl.

8. A compound as claimed in claim 1, wherein

the group $\underline{\cdots}$ is unsaturated (=),
A is the group -CH=,
$R^1$ is 3,3-dimethylbutyl, and
$R^2$ is tert-butyl.

9. A compound as claimed in claim 1, wherein

the group $\underline{\cdots}$ is saturated (-),
A is the group $-CH_2-$,
$R^1$ is 3,3-dimethylbutyl, and
$R^2$ is tert-butyl.

## Revendications

1. Pipérazines N, N′-disubstituée(s), de formule générale I

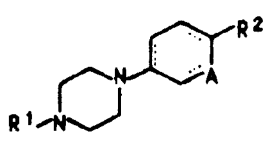

(I)

dans laquelle le groupe = est insaturé (=) ou saturé (-) et les substituants $R^1$, $R^2$ et A ont les significations suivantes :
A = -, -CH=, $-CH_2-$, $-CH_2-CH_2-$,
$R^1$, alkyle en C 2-C 20, alcényle en C 3-C 20, alcynyle en C 3-C 20, cycloalkyle en C 4-C 12, cycloalcényle en C 4-C 12, alkyle-cyclo=alkyle en C 4-C 20, cycloalcénylalkyle en C 4-C 20, alkyl-cycloalcényle en C 4-C 20, bicycloalkyle en C 9-C 11, bicycloalkylalkyle en C 10-C 15, alkyl-bicycloalkyle en C 10-C 15, ces restes étant éventuellement substitués par hydroxy, 1 à 3 atomes d'halogène, alcoxy en C 1-C 5 ou trialkylsilyle en C 3-C 9,
$R^1$, en outre, hétérocycloalkyle à 5 à 7 chaînons, contenant 1 ou 2 hétéro-atomes choisis dans le groupe oxygène et/ou soufre, hétérocycloalkylméthyle à 5 à 7 chaînons, comportant 1 ou 2 hétéroatomes choisi dans le groupe oxygène et/ou sufre, hétérocyclo=alkylméthyle à 5 à 7 chaînons, substitué par alkyle en C 1-C 8 et contenant 1 ou 2 hétéroatomes choisis dans le groupe oxygène et/ou soufre,
$R^2$, alkyle ramifié, en C 3-C 10, alcoxy ramifié, en C 3-C 8, triméthyl-silyle, cycloalkyle en C 3-C 7, alkylthio en C 3-C 8 et son sel phytophysiologiquement acceptable.

2. Procédé de préparation des composés selon la revendication 1, caractérisé par le fait que l'on fait réagir, dans des conditions basiques,

a) le composé II

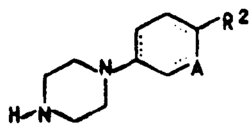

(II)

dans des conditions basiques, avec un composé de formule $R^1$-X, dans laquelle le substituant X représente un reste séparable en milieu basique, ou

b) le composé II avec un composé carbonyle III

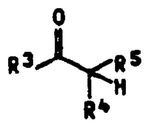

(III)

les restes $R^3$ à $R^5$ étant définis de manière à ce que le reste IV, dans sa totalité, corresponde au reste $R^1$

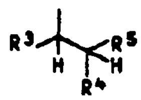

(IV)

$b_1$) dans des conditions réductrices, ou

$b_2$) pour obtenir une énamine et on hydrogène celle-ci par voie catalytique ou on la réduit avec de l'acide formique suivant Leukart-Wallach.

3. Fongicide contenant, outre des véhicules usuels, une pipérazine de formule générale I

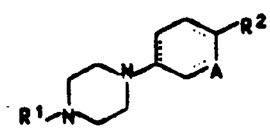

(I)

dans laquelle le groupe = est insaturé (=) ou saturé (-) et les substituants $R^1$, $R^2$ et A ont les significations suivantes :

A = -, -CH=, $-CH_2-$, $-CH_2-CH_2-$,

$R^1$, alkyle en C 2-C 20, alcényle en C 3-C 20, alcynyle en C 3-C 20, cycloalkyle en C 4-C 12, cycloalcényle en C 4-C 12, alkyle-cyclo=alkyle en C 4-C 20, cycloalcénylalkyle en C 4-C 20, alkyl-cycloalcényle en C 4-C 20, bicycloalkyle en C 9-C 11, bicycloalkylalkyle en C 10-C 15, alkyl-bicycloalkyle en C 10-C 15, ces restes étant éventuellement substitués par hydroxy, 1 à 3 atomes d'halogène, alcoxy en C 1-C 5 ou trialkylsilyle en C 3-C 9,

$R^1$, en outre, hétérocycloalkyle à 5 à 7 chaînons, contenant 1 ou 2 hétéro-atomes choisis dans le groupe oxygène et/ou soufre, hétérocycloalkylméthyle à 5 à 7 chaînons, comportant 1 ou 2 hétéroatomes choisi dans le groupe oxygène et/ou sufre, hétérocyclo=alkylméthyle à 5 à 7 chaînons, substitué par alkyle en C 1-C 8 et contenant 1 ou 2 hétéroatomes choisis dans le groupe oxygène et/ou soufre,

$R^2$, alkyle ramifié, en C 3-C 10, alcoxy ramifié, en C 3-C 8, triméthyl=silyle, cycloalkyle en C 3-C 7, alkylthio en C 3-C 8 et son sel phytophysiologiquement acceptable.

4. Utilisation des composés selon la revendication 1 comme fongicides.

5. Procédé de lutte contre les champignons, caractérisé par le fait que l'on traite les champignons ou les matériaux, plantes, semences ou sols menacés par l'attaque par les champignons, avec une pipérazine N, N'-disubstituée(s), de formule générale I

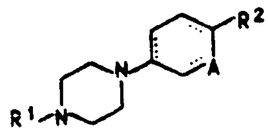

(I)

dans laquelle le groupe = est insaturé (=) ou saturé (-) et les substituants $R^1$, $R^2$ et A ont les significations suivantes :

A = -, -CH=, $-CH_2-$, $-CH_2-CH_2-$,

$R^1$, alkyle en C 2-C 20, alcényle en C 3-C 20, alcynyle en C 3-C 20, cycloalkyle en C 4-C 12, cycloalcényle en C 4-C 12, alkyle-cyclo=alkyle en C 4-C 20, cycloalcénylalkyle en C 4-C 20, alkyl-cycloalcényle en C 4-C 20, bicycloalkyle en C 9-C 11, bicycloalkylalkyle en C 10-C 15, alkyl-bicycloalkyle en C 10-C 15, ces restes étant éventuellement substitués par hydroxy, 1 à 3 atomes d'halogène, alcoxy en C 1-C 5 ou trialkylsilyle en C 3-C 9,

$R^1$, en outre, hétérocycloalkyle à 5 à 7 chaînons, contenant 1 ou 2 hétéro-atomes choisis dans le groupe oxygène et/ou soufre, hétérocycloalkylméthyle à 5 à 7 chaînons, comportant 1 ou 2 hétéroatomes choisi dans le groupe oxygène et/ou sufre, hétérocyclo-alkylméthyle à 5 à 7 chaînons, substitué par alkyle en C 1-C 8 et contenant 1 ou 2 hétéroatomes choisis dans le groupe oxygène et/ou soufre,

$R^2$, alkyle ramifié, en C 3-C 10, alcoxy ramifié, en C 3-C 8, triméthyl-silyle, cycloalkyle en C 3-C 7, alkylthio en C 3-C 8 et son sel phytophysiologiquement acceptable.

6. Composé selon la revendication 1, caractérisé par le fait que
le groupe = est insaturé (=),
A représente le groupe -CH=,
$R^1$, 3,3-diméthylcyclohexyle et
$R^2$, tert-butyle.

7. Composé selon la revendication 1, caractérisé par le fait que
le groupe = est saturé (-),
A représente le groupe $-CH_2-$,
$R^1$, 3,3-diméthylcyclohexyle et
$R^2$, tert-butyle.

8. Composé selon la revendication 1, caractérisé par le fait que
le groupe = est insaturé (=),
A représente le groupe -CH=,
$R^1$, 3,3-diméthylbutyle et
$R^2$, tert-butyle.

9. Composé selon la revendication 1, caractérisé par le fait que
le groupe = est saturé (-),
A représente le groupe $-CH_2-$,
$R^1$, 3,3-diméthylbutyle et
$R^2$, tert-butyle.